# EUROPEAN PATENT SPECIFICATION

(11) **EP 3 876 846 B1**
(45) Date of publication and mention of the grant of the patent: **28.01.2026**
(21) Application number: 19813956.0
(22) Date of filing: 07.11.2019
(51) Int. Cl.: A61B 17/11, A61B 17/12

(54) **SHEATH FOR USE IN PERFORMING SURGICAL VASCULAR ANASTOMOTIC PROCEDURES, AND APPLICATIONS THEREOF**
HÜLLE ZUR VERWENDUNG BEI DER DURCHFÜHRUNG VON CHIRURGISCHEN VASKULÄREN ANASTOMOTISCHEN VERFAHREN UND ANWENDUNGEN DAFÜR
GAINE DESTINÉE À ÊTRE UTILISÉE DANS LA RÉALISATION DE PROCÉDURES CHIRURGICALES D'ANASTOMOSE VASCULAIRE, ET SES APPLICATIONS

(30) Priority: 07.11.2018 US 201862756871 P
(43) Date of publication of application: 15.09.2021
(73) Proprietor: Vascular Graft Solutions Ltd., 6971921 Tel Aviv (IL)
(72) Inventor: ORION, Eyal, 52960 Ramat Efal (IL); HARARI, Boaz, 55900 Geney Tikva (IL)
(74) Representative: Kutzenberger Wolff & Partner
(86) International application number: PCT/IL2019/051220
(87) International publication number: WO 2020/095306

(56) References cited:
- US-A- 5 591 129
- US-A1- 2010 010 519
- US-A1- 2011 319 905
- US-A1- 2013 006 281
- US-A1- 2014 200 441
- US-B1- 6 280 413

## Description

### RELATED APPLICATION

This application claims the benefit of priority under 35 USC 119(e) of U.S. Provisional Patent Application No. 62/756,871, filed November 07, 2018, entitled "Sheath For Use In Performing Surgical Vascular Anastomotic Procedures, and Methods Thereof".

### FIELD OF THE INVENTION

The present invention, in some embodiments thereof, relates to techniques in performing surgical vascular anastomotic procedures, and more particularly, but not exclusively, to a sheath for use in performing surgical vascular anastomotic procedures, and applications thereof. Some embodiments of the present invention are particularly applicable for use in 'clampless' types of (end-to-side) surgical vascular anastomotic procedures, for performing coronary artery bypass grafting (CABG).

### BACKGROUND OF THE INVENTION

In performing surgical vascular anastomotic procedures, employed, for example, in coronary artery bypass grafting (CABG), a sheath is used for externally covering, enclosing, and holding a (self-expandable) anastomotic hole sealing assembly or element in a non-activated, collapsed configuration. In such procedures, the sheath (with the collapsed hole sealing assembly or element therein) is guided and positioned into a blood vessel lumen (e.g., of an aorta), by directing the sheath through a small (needle or syringe sized) hole or incision in a blood vessel wall. While inside the blood vessel lumen, the sheath is removed from the hole sealing assembly or element so as to facilitate conversion and activation of the hole sealing assembly or element from the collapsed configuration to an activated, self-expanded configuration. Thereafter, an anastomotic procedure (e.g., anastomosis) is performed on the host blood vessel with a blood vessel graft, followed by pulling the sheath back onto the hole sealing assembly or element, so as to return the hole sealing assembly or element to a non-activated, collapsed configuration, prior to removal of the sheath (with the collapsed hole sealing assembly or element therein) from the blood vessel, and ultimately, from the subject, followed by completing the anastomotic procedure.

Exemplary techniques (apparatuses, methods) involving such use of sheaths are disclosed in US 2006/0079915 A1 (Chin, et al.), and in same applicant/assignee WO 2018/203237 A1 (Nov. 11, 2018).

Additional teachings about sheaths and sheath-like devices, and their uses, in various anastomotic procedures are as follows.

US 2010/0010519 A1 (Stopek, et al.) relates to a sheath for use with an anastomosis to prevent fluid leaks, and more particularly to a sheath made with at least one biodegradable portion (attached proximal to an anastomotic site) and at least one non-biodegradable portion. The sheath prevents anastomotic leakage of tissue luminal contents into the surrounding cavity. A method for using the sheath is also disclosed.

US 2013/0006281 A1 (Golden, et al.) teaches about various aspects and embodiments of an apparatus and methods for performing an anastomosis. For example, the apparatus may be used to perform a single or multiple anastomosis with the ability of maintaining fluid flow (e.g., blood) through the anastomosis vessel.

US 6,280,413 (Clark, et al.) teaches about a thrombolytic filtration and drug delivery catheter, and more particularly, a thrombolytic filtration and drug delivery catheter comprising longitudinal ribs which are advanced to the desired site in a compressed position and can be released to a deployed position at the desired site, flaring to a diameter greater than the diameter of the shaft. The ribs can be compressed by a sleeve or thread, for example. Drugs or other agents can be delivered through lumens in the shaft and ribs, out through ports in the ribs. Preferably, the ports are positioned to deliver the drugs or other agents between the ribs and within the region defined by the ribs.

US 2014/0200441 A1 (Potter, et al.) teaches about a catheter tip comprising a tip electrode comprising a ledge feature, and a center cavity and a manifold assembly comprising a fluid lumen manifold and a stop tube. The stop tube can be coupled to the fluid lumen manifold and configured to abut the ledge feature such that a distal end of the fluid lumen manifold extends a pre-determined distance into the center cavity of the tip electrode. The fluid lumen manifold comprises a plurality of side holes sized and configured to distribute an irrigant to the tip electrode. The catheter tip comprises a flexible tip electrode.

US5591129A relates to angioplasty and, in particular, to perfusion balloon angioplasty catheters.

In spite of these and other teachings in the field and art of the invention, there is need for developing and implementing new or/and improved sheaths for use in performing surgical vascular anastomotic procedures.

### SUMMARY OF THE INVENTION

The invention is defined by the independent claim. The dependent claims define advantageous embodiments. The present invention, in some embodiments thereof, relates to a sheath for use in performing surgical vascular anastomotic procedures, and applications thereof. Some embodiments of the present invention are particularly applicable for use in 'clampless' types of (end-to-side) surgical vascular anastomotic procedures, for performing coronary artery bypass grafting (CABG).

In exemplary embodiments, the sheath includes a sheath main portion configured as a tubular member and having a sheath main portion distal end and a sheath main portion proximal end, wherein the sheath main portion proximal end is open. The sheath further includes a sheath distal end portion, continuous with the sheath main portion distal end, and including at least one intra-sheath fluid flow passageway, whereby each one of the at least one intra-sheath fluid flow passageway extends and passes entirely through the inside of the sheath distal end portion, via two differently located parts of the sheath distal end portion. Each intra-sheath fluid (blood, water, air) flow passageway (tunnel) inside of the sheath distal end portion is configured (shaped and sized) so as to facilitate *continuous* flow of blood vessel lumen fluids (blood, water, air) through the overall sheath, especially, flow of blood vessel lumen fluids that enter through the sheath (open) proximal end and exit through the sheath (open) distal end portion.

In exemplary embodiments, the sheath includes a *single* intra-sheath fluid (blood, water, air) flow passageway (tunnel) in the sheath distal end portion, or includes a *plurality* of exemplary intra-sheath fluid (blood, water, air) flow passageways (tunnels) in the sheath distal end portion. In such exemplary embodiments, the sheath has either a *cylindrical* (cylindrically shaped) proximal end portion or a *conical* (conically shaped) proximal end portion. In exemplary embodiments, the *conical* proximal end portion may better facilitate pulling of the sheath back onto an activated, self-expanded hole sealing assembly or element, so as to return the hole sealing assembly or element to a non-activated, collapsed configuration, prior to removal of the sheath (with the collapsed hole sealing assembly or element therein) from the blood vessel, and ultimately, from a subject undergoing an anastomotic procedure.

In embodiments according to the invention, the sheath distal end portion also includes an intra-sheath control wire anchoring and fixing pocket (cavity), and a sheath control wire passageway (tunnel). In exemplary embodiments, one of the intra-sheath fluid flow passageways (tunnels) is also structured and functional as a combination of: (i) the intra-sheath control wire anchoring and fixing pocket (cavity) and (ii) the sheath control wire passageway (tunnel), inside the sheath distal end portion. The sheath also includes a sheath flexible control wire, extending along and through the inside of the sheath, and being anchored and fixedly connected to the inside of the sheath distal end portion, via the intra-sheath control wire anchoring and fixing pocket (cavity).

According to an aspect of some embodiments of the present invention, there is provided a sheath for use in performing surgical vascular anastomotic procedures, the sheath comprising: a sheath main portion configured as a tubular member and including a sheath main portion distal end and a sheath main portion proximal end, the sheath main portion proximal end is open; and a sheath distal end portion, continuous with the sheath main portion distal end, and including at least one intra-sheath fluid flow passageway, whereby each one of the at least one intra-sheath fluid flow passageway extends and passes entirely through the inside of the sheath distal end portion, via two differently located parts of the sheath distal end portion; whereby each intra-sheath fluid flow passageway facilitates continuous flow of blood vessel lumen fluids through the sheath.

According to some embodiments of the invention, the sheath main portion and the sheath distal end portion are configured as a single, monolithic structure, wherein the sheath main portion and the sheath distal end portion are integrally formed as a single continuous structure. According to some embodiments of the invention, the sheath main portion and the sheath distal end portion are configured as two individual, operably connectable, structures. According to some embodiments of the invention, the sheath distal end portion is configured with a proximal end portion that closely fits into the tubular distal end portion of the sheath main portion. According to some embodiments of the invention, the sheath main portion has a conical proximal end portion that includes the sheath main portion open proximal end.

According to some embodiments of the invention, one of the at least one intra-sheath fluid flow passageway extends and passes entirely through the middle or center, proximal-distal, longitudinal axis inside of the sheath distal end portion, via two oppositely, diametrically opposed, and centrally, located parts of the sheath distal end portion.

According to embodiments of the invention, the sheath distal end portion also includes an intra-sheath control wire anchoring and fixing pocket.

According to some embodiments of the invention, the intra-sheath control wire anchoring and fixing pocket is tubular and includes two portions, a first portion and a second portion, wherein the inner diameter of the first portion is less than the inner diameter of the second portion. According to some embodiments of the invention, the sheath distal end portion is also configured with a sheath control wire passageway. According to some embodiments of the invention, the sheath control wire passageway has an inner diameter that is larger than the inner diameter of each of the first and second portions of the intra-sheath control wire anchoring and fixing pocket. According to some embodiments of the invention, the sheath further includes a sheath control wire, extending along and through the inside of the sheath, and being anchored and fixedly connected to the inside of the sheath distal end portion, via the intra-sheath control wire anchoring and fixing pocket.

According to some embodiments of the invention, at least one intra-sheath fluid flow passageway extends and passes entirely through a non-middle or off-center, proximal-distal, longitudinal axis inside of the sheath distal end portion, via two oppositely, diametrically opposed, and non-centrally, located parts of the sheath distal end portion.

According to some embodiments of the invention, at least one intra-sheath fluid flow passageway radially, orthogonally to proximal - distal directions, extends and passes entirely through the inside of the sheath distal end portion, via a pair of two oppositely located, diametrically opposed, parts of the circumferential periphery surface of the sheath distal end portion.

According to some embodiments of the invention, the sheath distal end portion also includes an intra-sheath control wire anchoring and fixing pocket. According to some embodiments of the invention, the intra-sheath control wire anchoring and fixing pocket is tubular and includes two portions, a first portion and a second portion, wherein the inner diameter of the first portion is less than the inner diameter of the second portion. According to some embodiments of the invention, the sheath distal end portion is also configured with a sheath control wire passageway. According to some embodiments of the invention, the sheath control wire passageway has an inner diameter that is larger than the inner diameter of each of the first and second portions of the intra-sheath control wire anchoring and fixing pocket. According to some embodiments of the invention, the sheath further includes a sheath control wire, extending along and through the inside of the sheath, and being anchored and fixedly connected to the inside of the sheath distal end portion, via the intra-sheath control wire anchoring and fixing pocket.

According to some embodiments of the invention, at least one the intra-sheath fluid flow passageway radially, non-orthogonally to proximal - distal directions, extends and passes entirely through the inside of the sheath distal end portion, via a pair of two oppositely located, diametrically opposed, parts of the circumferential periphery surface of the sheath distal end portion.

According to some embodiments of the invention, the sheath distal end portion is configured as a member having a cylindrical base section and a conical top section thereupon, and wherein at least one intra-sheath fluid flow passageway extends and passes entirely through the inside of the cylindrical base section of the sheath distal end portion.

According to some embodiments of the invention, the intra-sheath fluid flow passageway radially, orthogonally to proximal - distal directions, extends and passes entirely through the inside of the cylindrical base section of the sheath distal end portion.

According to some embodiments of the invention, the sheath distal end portion is configured as a member having a cylindrical base section and a conical top section thereupon, and wherein at least one intra-sheath fluid flow passageway extends and passes entirely through the inside of the conical top section of the sheath distal end portion.

According to some embodiments of the invention, the intra-sheath fluid flow passageway radially, orthogonally to proximal - distal directions, extends and passes entirely through the inside of the conical top section of the sheath distal end portion.

According to some embodiments of the invention, the sheath main portion is made of a material selected from the group consisting of non-metallic materials, metallic materials, polymeric materials, composite materials, and a combination thereof. According to some embodiments of the invention, the sheath main portion is made of a non-metallic, polymeric material. According to some embodiments of the invention, the non-metallic, polymeric material is polyethylene plastic.

According to some embodiments of the invention, the sheath main portion includes a porous or/and a non-porous structure. According to some embodiments of the invention, the porous structure is in a form of a braid, having struts and holes or spaces therebetween. According to some embodiments of the invention, the porous structure is made of a material selected from the group consisting of non-metallic materials, metallic materials, polymeric materials, composite materials, and a combination thereof.

According to some embodiments of the invention, the sheath main portion is configured as a braid. According to some embodiments of the invention, the braid is made of a non-metallic, polymeric material. According to some embodiments of the invention, the polymeric material is polyethylene (PET) plastic.

According to some embodiments of the invention, the sheath main portion external or outer surface is coated with a coating material. According to some embodiments of the invention, the coating material is selected from the group consisting of polyether block amides. According to some embodiments of the invention, the polyether block amide is a Pebax^{®} polymeric resin. According to some embodiments of the invention, the sheath main portion internal or inner surface is coated with a coating material. According to some embodiments of the invention, the coating material is selected from the group consisting of polytetrafluoroethylenes (PTFE).

According to some embodiments of the invention, the sheath distal end portion is made of a material selected from the group consisting of non-metallic materials, metallic materials, polymeric materials, composite materials, and a combination thereof. According to some embodiments of the invention, the sheath distal end portion is configured as a member having a cylindrical base section and a conical top section thereupon, and wherein the cylindrical base section and the conical top section are made of a same material, or of different materials. According to some embodiments of the invention, the sheath distal end portion is made of a single material being a metallic material. According to some embodiments of the invention, the metallic material is stainless steel.

According to some embodiments of the invention, the sheath distal end portion is made of a single material being a non-metallic, polymeric material. According to some embodiments of the invention, the polymeric material is polyether ether ketone.

All technical or/and scientific words, terms, or/and phrases, used herein have the same or similar meaning as commonly understood by one of ordinary skill in the art to which the invention pertains, *unless otherwise specifically defined or stated herein.* Exemplary embodiments of methods (steps, procedures), apparatuses (devices, systems, components thereof), equipment, and materials, illustratively described herein are exemplary and illustrative only and are not intended to be necessarily limiting. Although methods, apparatuses, equipment, and materials, equivalent or similar to those described herein can be used in practicing or/and testing embodiments of the invention, exemplary methods, apparatuses, equipment, and materials, are illustratively described below. In case of conflict, the patent specification, including definitions, will control.

### BRIEF DESCRIPTION OF THE SEVERAL VIEWS OF THE DRAWINGS

Some embodiments of the present invention are herein described, by way of example only, with reference to the accompanying drawings. With specific reference now to the drawings in detail, it is stressed that the particulars shown are by way of example and for purposes of illustrative description of some embodiments of the present invention. In this regard, the description taken together with the accompanying drawings make apparent to those skilled in the art how some embodiments of the present invention may be practiced.

In the drawings:

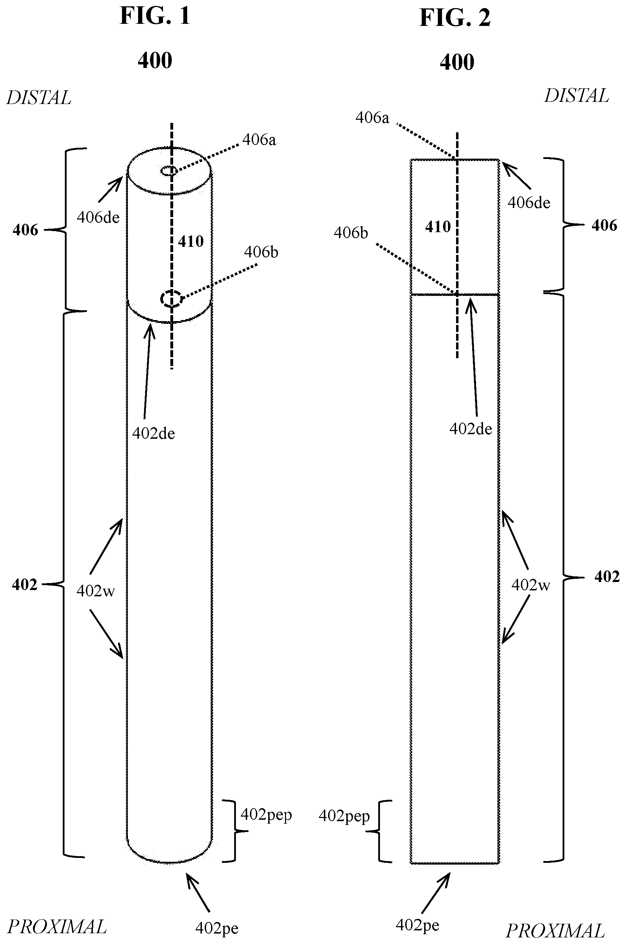

FIGs. 1 and 2 are schematic perspective and side views, respectively, of an exemplary embodiment of the sheath (having a *cylindrical* proximal end portion), highlighting external appearance of a *single* exemplary intra-sheath fluid (blood, water, air) flow passageway (tunnel) in the sheath distal end portion, in accordance with some embodiments of the invention;

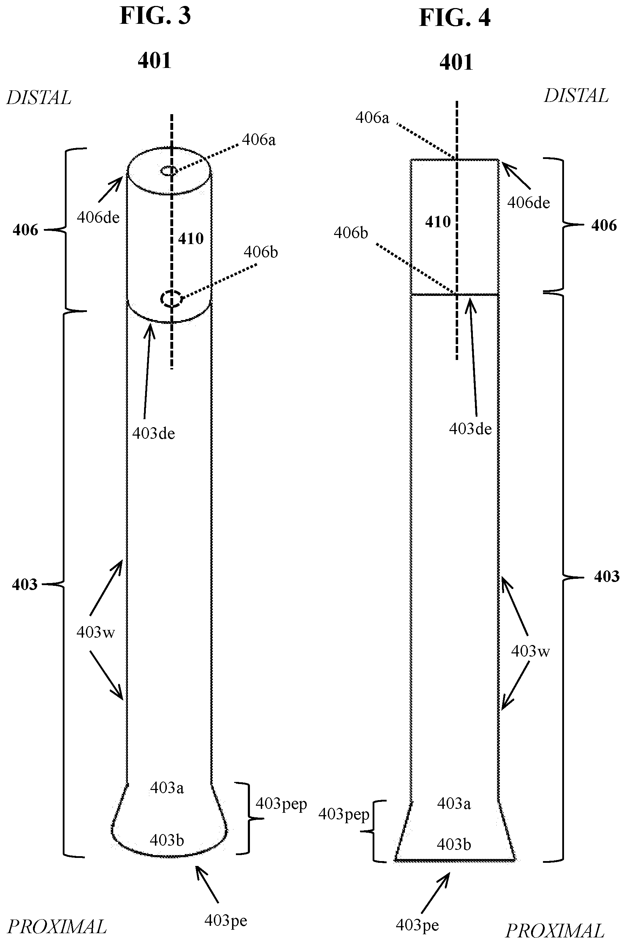

FIGs. 3 and 4 are schematic perspective and side views, respectively, of another exemplary embodiment of the sheath (having a *conical* proximal end portion), highlighting external appearance of a *single* exemplary intra-sheath fluid (blood, water, air) flow passageway (tunnel) in the sheath distal end portion, in accordance with some embodiments of the invention;

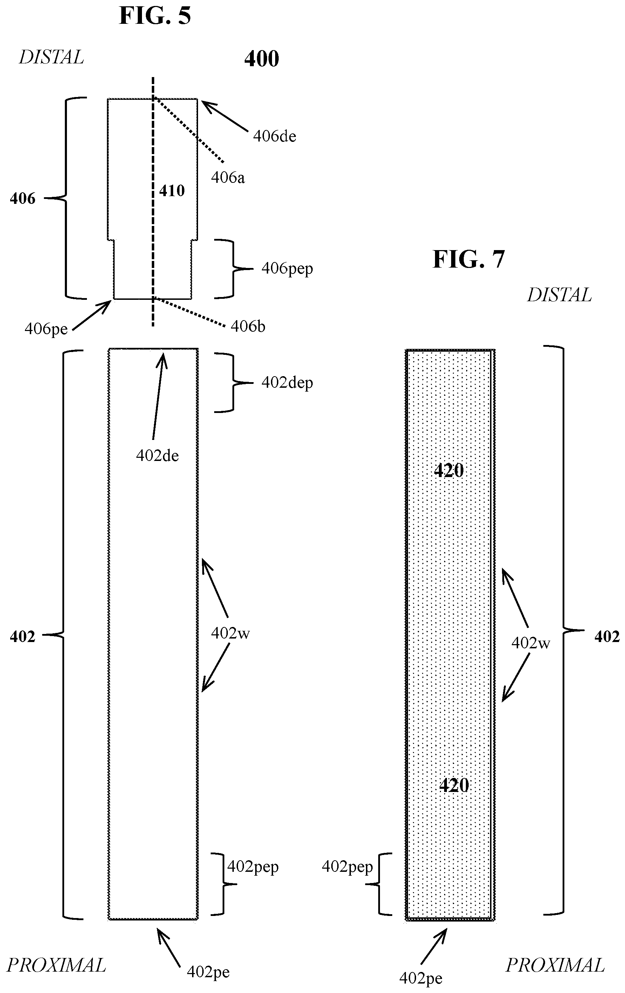

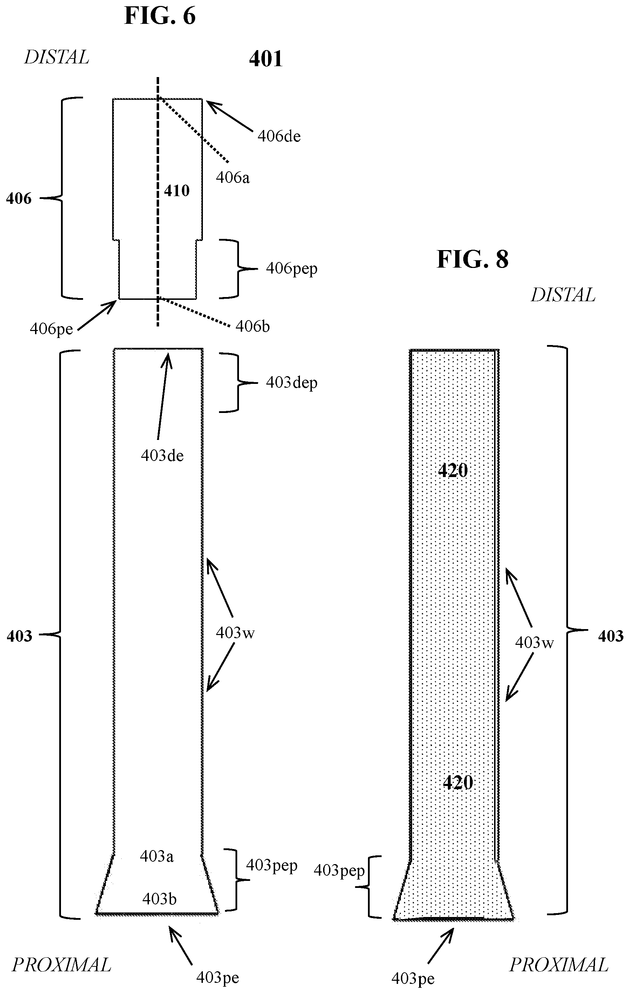

FIGs. 5 and 6 are schematic exploded side views of exemplary embodiments of the sheath, having a *cylindrical* proximal end portion [FIG. 5], and a *conical* proximal end portion [FIG. 6], respectively, highlighting the sheath main portion (tubular member) and the sheath distal end portion configured as two individual, operably connectable structures (components), in accordance with some embodiments of the invention;
FIGs. 7 and 8 are schematic side views of exemplary embodiments of the sheath main portion (shaft), having a *cylindrical* proximal end portion [FIG. 7], and a *conical* proximal end portion [FIG. 8], respectively, highlighting a coating upon the external surface of the sheath main portion (tubular member), in accordance with some embodiments of the invention;

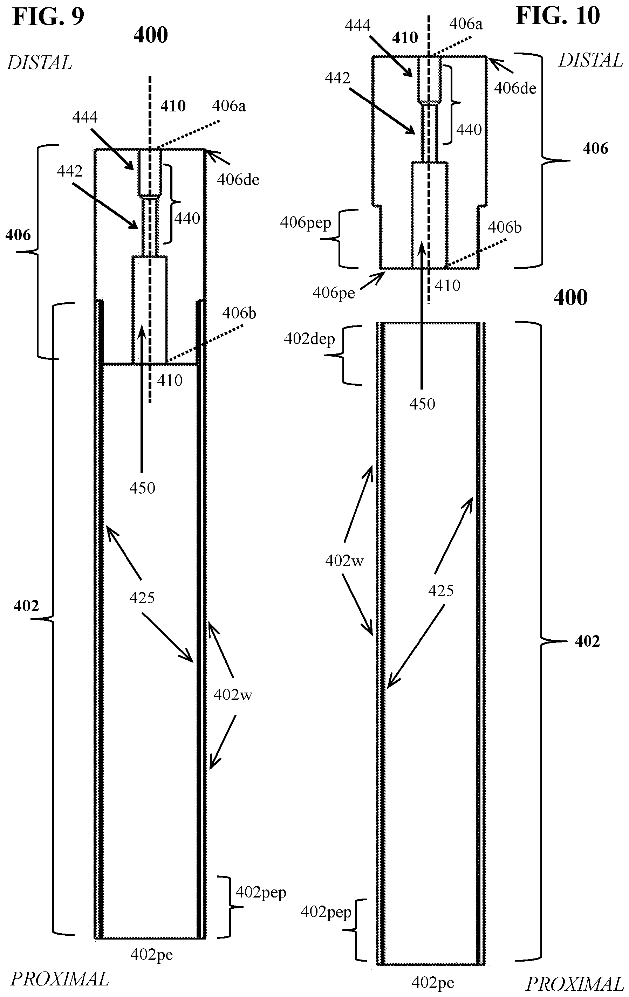

FIGs. 9 and 10 are schematic cross-sectional, and exploded cross-sectional, side views, respectively, of exemplary embodiments of the sheath, having a *cylindrical* proximal end portion, highlighting internal appearance and configuration of a *single* exemplary intra-sheath fluid (blood, water, air) flow passageway (tunnel) [also structured and functional as a combination of an exemplary intra-sheath control wire anchoring and fixing pocket (cavity) and an exemplary sheath control wire passageway (tunnel)], inside the sheath distal end portion, in accordance with some embodiments of the invention;

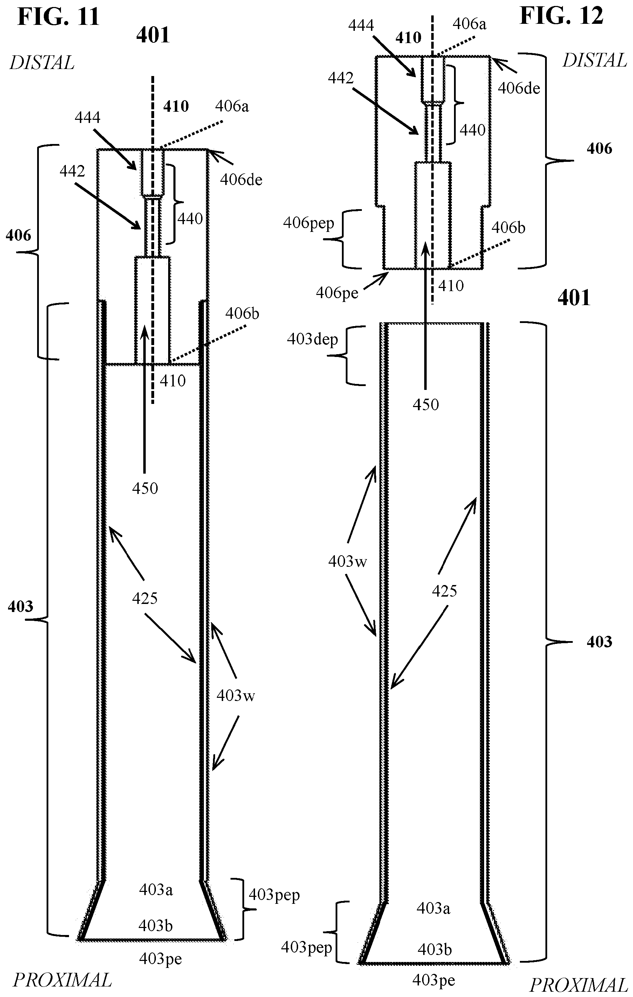

FIGs. 11 and 12 are schematic cross-sectional, and exploded cross-sectional, side views, respectively, of exemplary embodiments of the sheath, having a *conical* proximal end portion, highlighting internal appearance and configuration of a *single* exemplary intra-sheath fluid (blood, water, air) flow passageway (tunnel) [also structured and functional as a combination of an exemplary intra-sheath control wire anchoring and fixing pocket (cavity) and an exemplary sheath control wire passageway (tunnel)], inside the sheath distal end portion, in accordance with some embodiments of the invention;

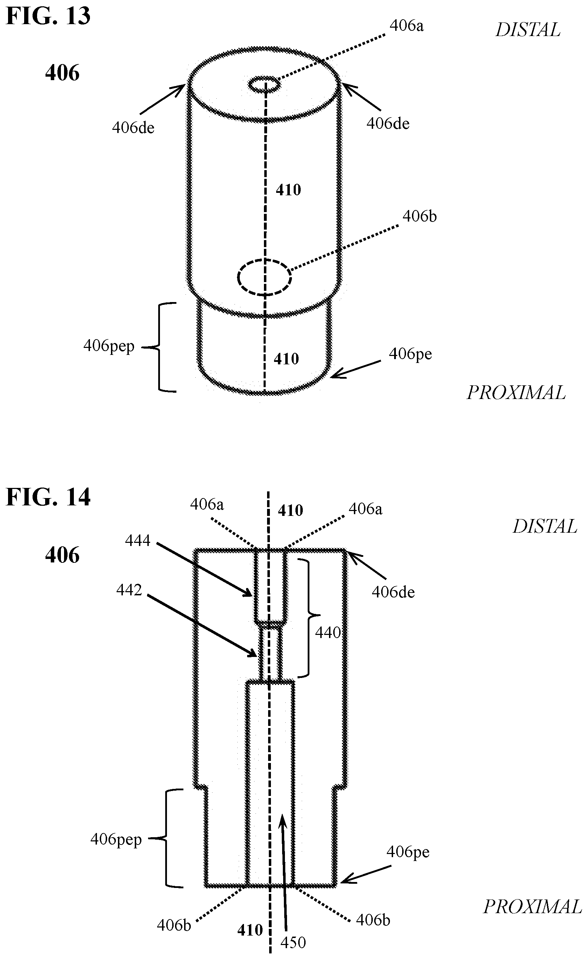

FIGs. 13 and 14 are a schematic close-up perspective view, and a schematic close-up cross-sectional side view, respectively, of an exemplary embodiment of the sheath distal end portion, in accordance with some embodiments of the invention;

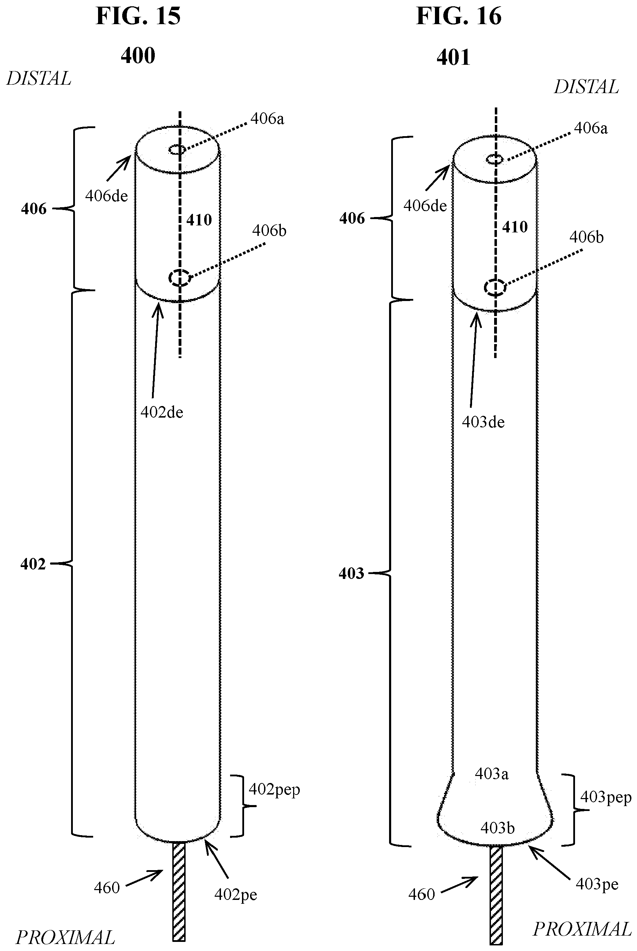

FIGs. 15 and 16 are schematic perspective views of exemplary embodiments of the sheath, having a *cylindrical* proximal end portion [FIG. 15], and a *conical* proximal end portion [FIG. 16], respectively, highlighting inclusion of an exemplary sheath flexible control wire extending along and through the inside of the sheath, in accordance with some embodiments of the invention;

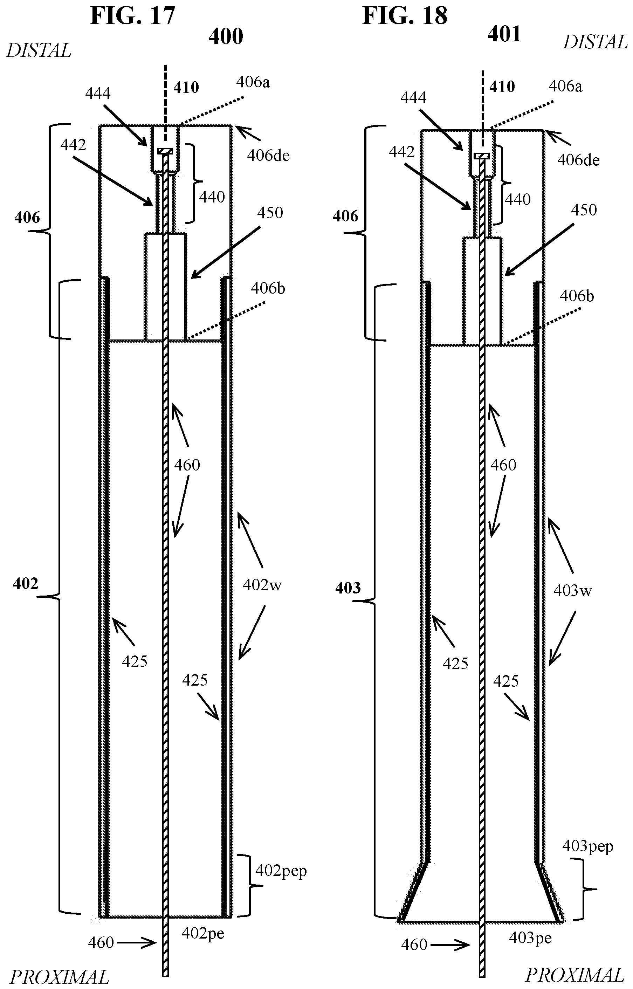

FIGs. 17 and 18 are schematic cross-sectional side views of exemplary embodiments of the sheath, having a *cylindrical* proximal end portion [FIG. 17], and a *conical* proximal end portion [FIG. 18], respectively, highlighting the sheath flexible control wire anchored and fixedly connected to the inside of the sheath distal end portion, in accordance with some embodiments of the invention;

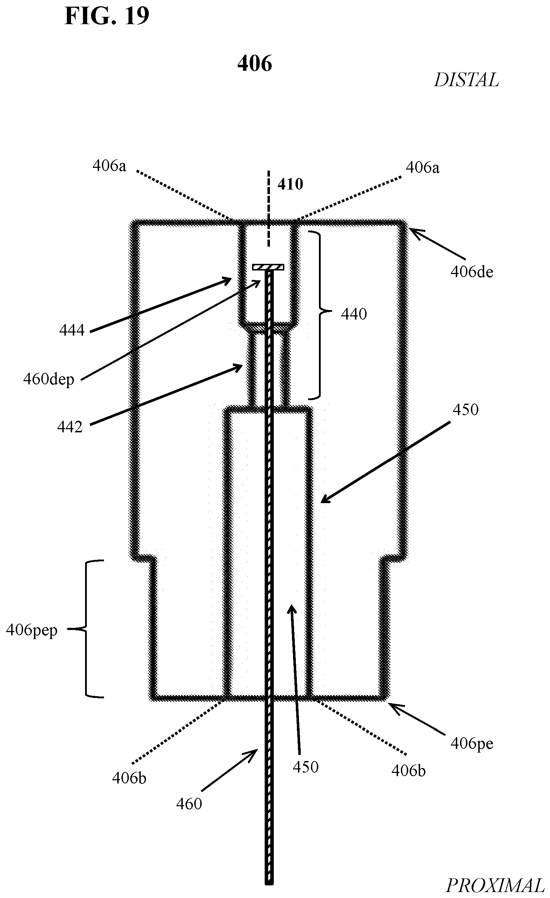

FIG. 19 is a schematic close-up cross-sectional side view of an exemplary embodiment of the sheath distal end portion, highlighting anchoring and fixed connection therein of the sheath flexible control wire, in accordance with some embodiments of the invention;

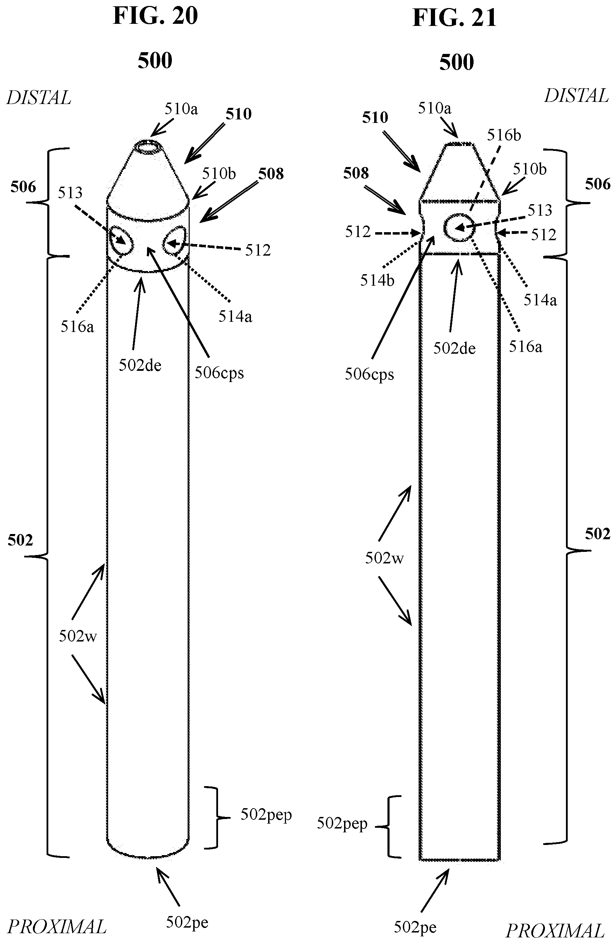

FIGs. 20 and 21 are schematic perspective and side views, respectively, of an exemplary embodiment of the sheath (having a *cylindrical* proximal end portion), highlighting external appearance of *a plurality* of exemplary intra-sheath fluid (blood, water, air) flow passageways (tunnels) in the sheath distal end portion, in accordance with some embodiments of the invention;

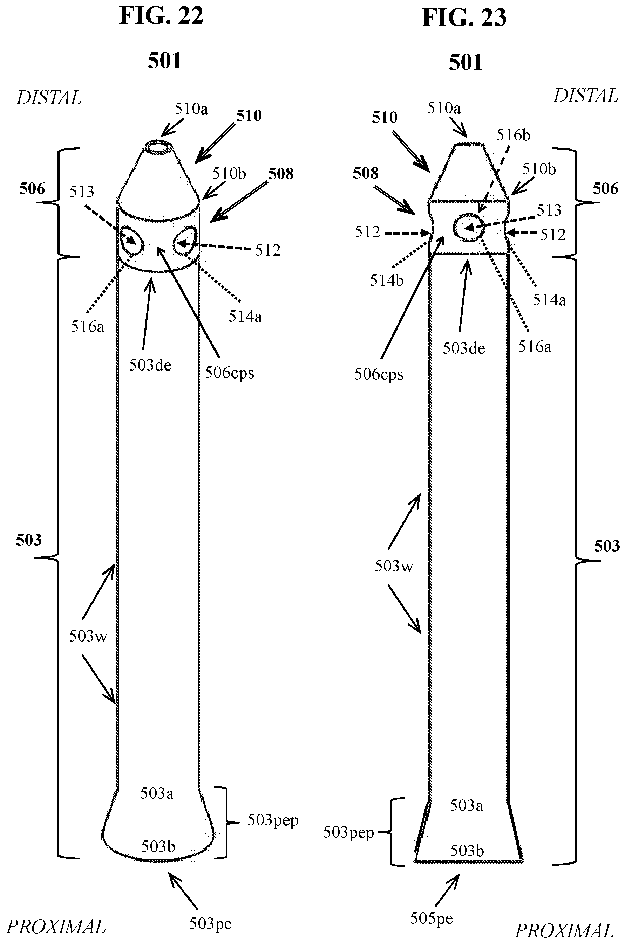

FIGs. 22 and 23 are schematic perspective and side views, respectively, of another exemplary embodiment of the sheath (having a *conical* proximal end portion), highlighting external appearance of a *plurality* of exemplary intra-sheath fluid (blood, water, air) flow passageways (tunnels) in the sheath distal end portion, in accordance with some embodiments of the invention;

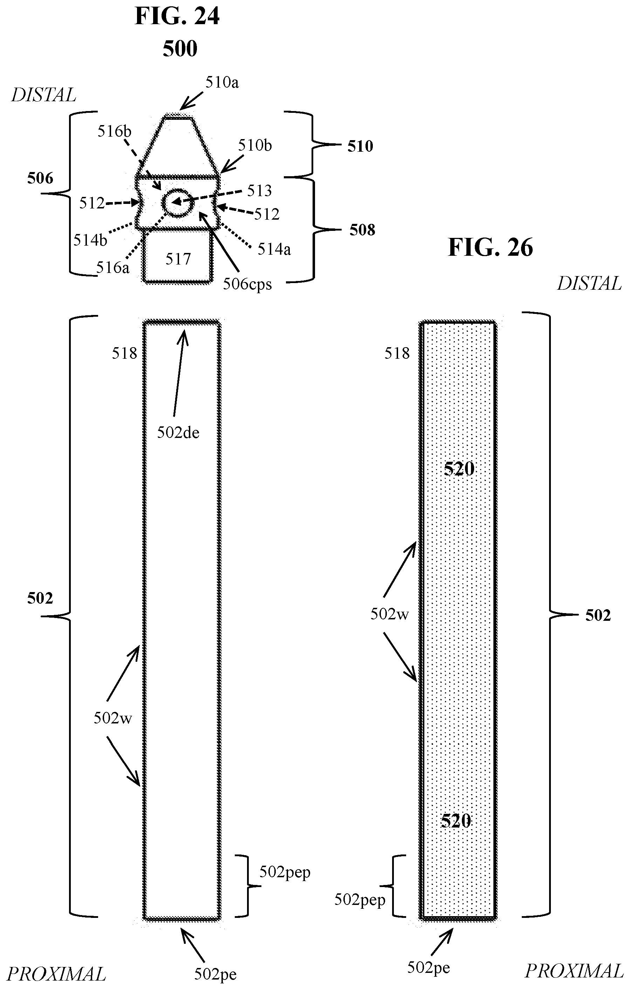

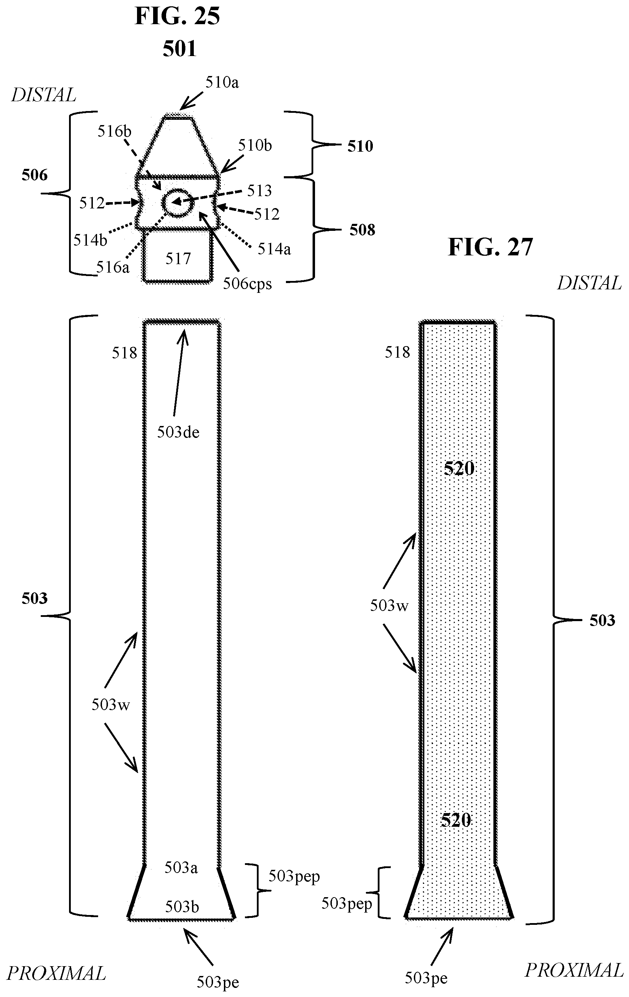

FIGs. 24 and 25 are schematic exploded side views of exemplary embodiments of the sheath, having a *cylindrical* proximal end portion [FIG. 24], and a *conical* proximal end portion [FIG. 25], respectively, highlighting the sheath main portion (tubular member) and the sheath distal end portion (including a cylindrical base and a conical top thereupon) configured as two individual, operably connectable structures (components), in accordance with some embodiments of the invention;
FIGs. 26 and 27 are schematic side views of exemplary embodiments of the sheath main portion (shaft), having a *cylindrical* proximal end portion [FIG. 26], and a *conical* proximal end portion [FIG. 27], respectively, highlighting a coating upon the external surface of the sheath main portion (tubular member), in accordance with some embodiments of the invention;

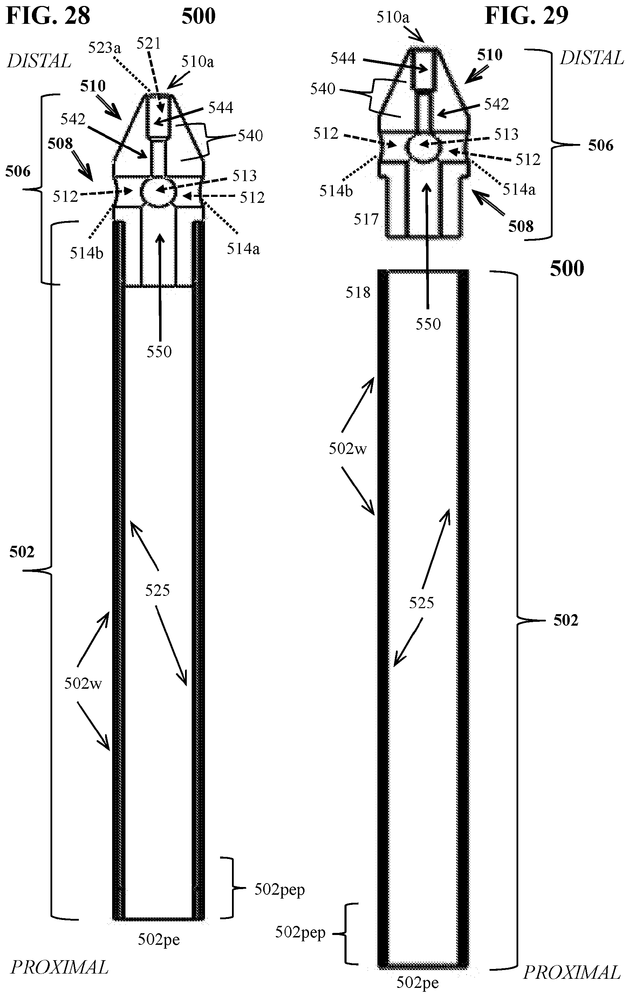

FIGs. 28 and 29 are schematic cross-sectional, and exploded cross-sectional, side views, respectively, of exemplary embodiments of the sheath, having a *cylindrical* proximal end portion, highlighting internal appearance and configuration of: (i) two exemplary intra-sheath fluid (blood, water, air) flow passageways (tunnels), (ii) an exemplary intra-sheath control wire anchoring and fixing pocket (cavity), and (iii) an exemplary sheath control wire passageway (tunnel), whereby the combination of (ii) and (iii) is also structured and functional as another exemplary intra-sheath fluid flow passageway (tunnel), inside the sheath distal end portion, in accordance with some embodiments of the invention;

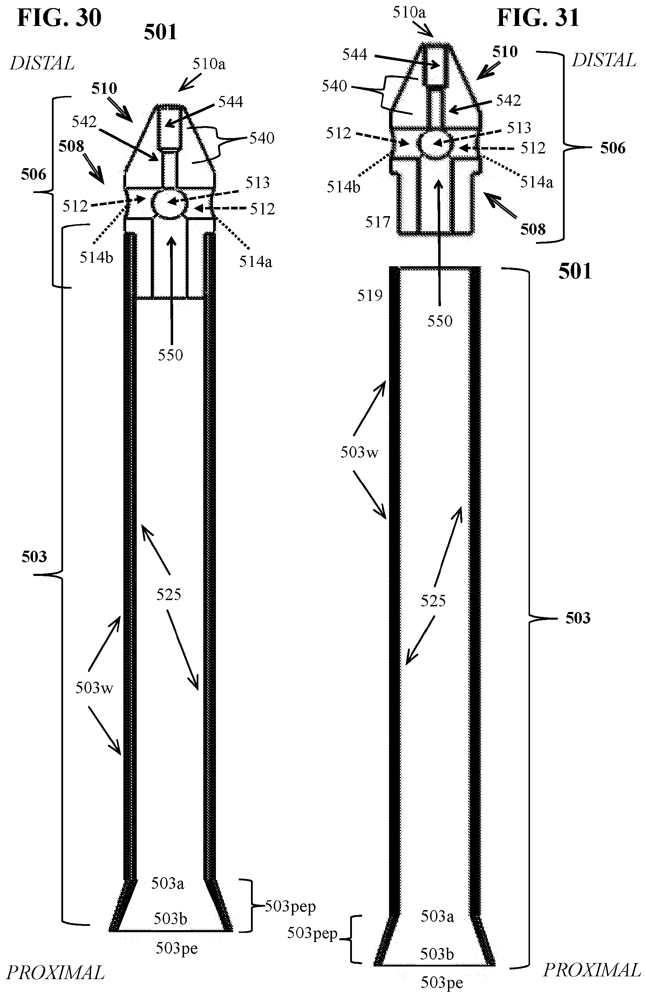

FIGs. 30 and 31 are schematic cross-sectional, and exploded cross-sectional, side views, respectively, of exemplary embodiments of the sheath, having a *conical* proximal end portion, highlighting internal appearance and configuration of: (i) two exemplary intra-sheath fluid (blood, water, air) flow passageways (tunnels), (ii) an exemplary intra-sheath control wire anchoring and fixing pocket (cavity), and (iii) an exemplary sheath control wire passageway (tunnel), whereby the combination of (ii) and (iii) is also structured and functional as another exemplary intra-sheath fluid flow passageway (tunnel), inside the sheath distal end portion, in accordance with some embodiments of the invention;

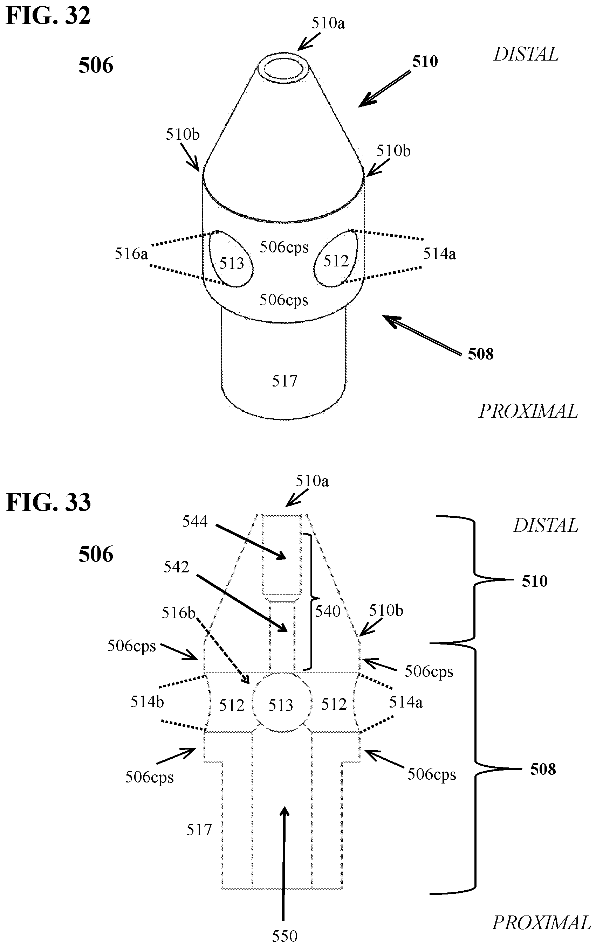

FIGs. 32 and 33 are a schematic close-up perspective view, and a schematic close-up cross-sectional side view, respectively, of an exemplary embodiment of the sheath distal end portion (including a cylindrical base and a conical top thereupon), in accordance with some embodiments of the invention;

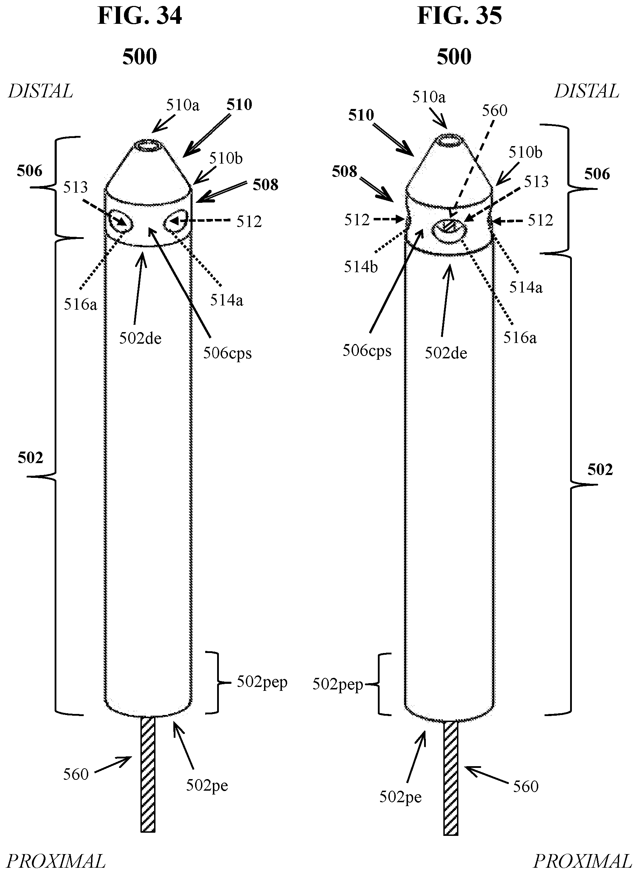

FIGs. 34 and 35 are schematic perspective views of an exemplary embodiment of the sheath (having a *cylindrical* proximal end portion), highlighting inclusion of an exemplary sheath flexible control wire extending along and through the inside of the sheath, in accordance with some embodiments of the invention;

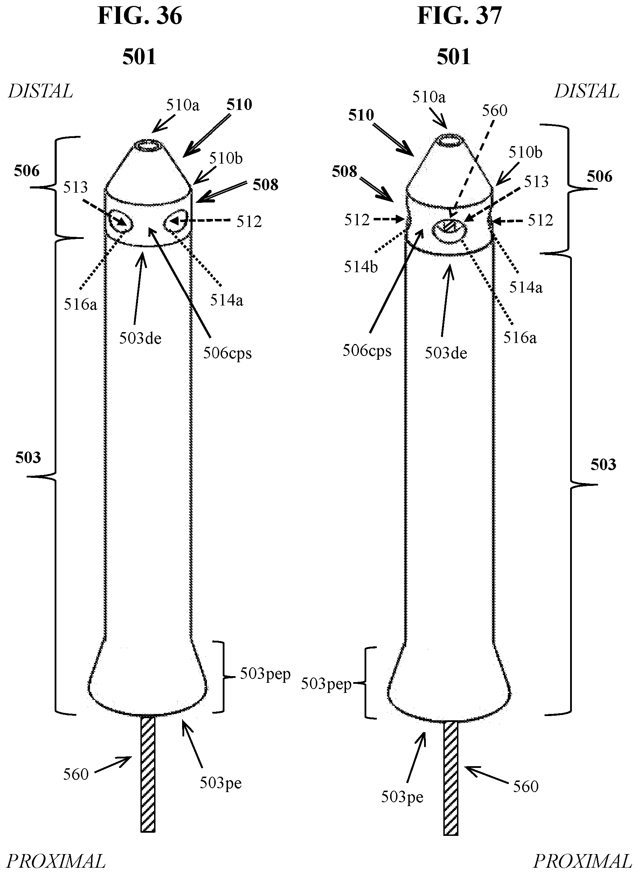

FIGs. 36 and 37 are schematic perspective views of an exemplary embodiment of the sheath (having a *conical* proximal end portion), highlighting inclusion of an exemplary sheath flexible control wire extending along and through the inside of the sheath, in accordance with some embodiments of the invention;

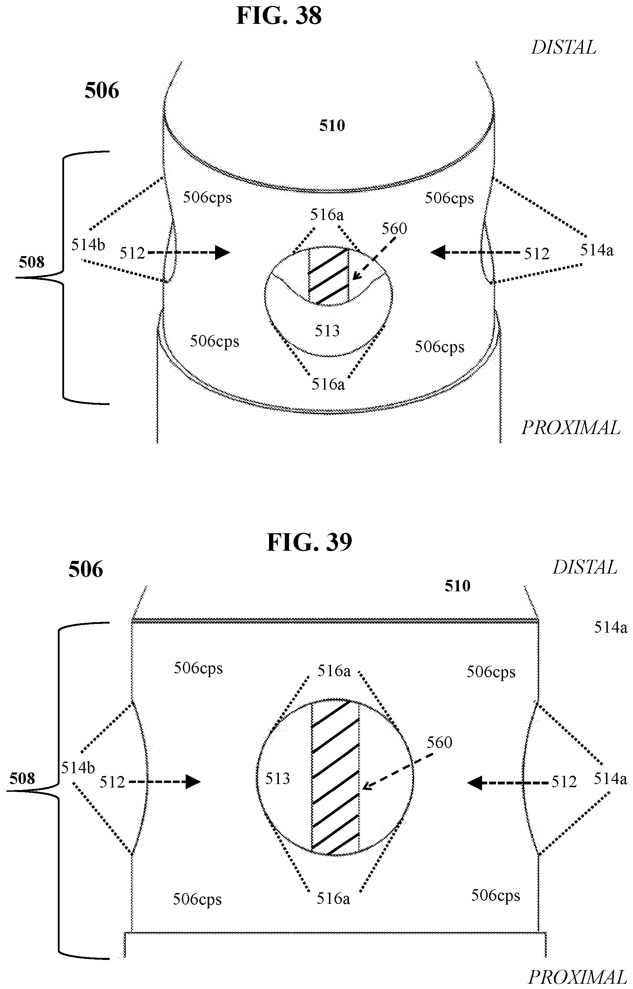

FIGs. 38 and 39 are a schematic close-up perspective view, and a schematic close-up side view, respectively, of an exemplary embodiment of part of the sheath distal end portion, highlighting configuration of the sheath flexible control wire therein, in accordance with some embodiments of the invention;

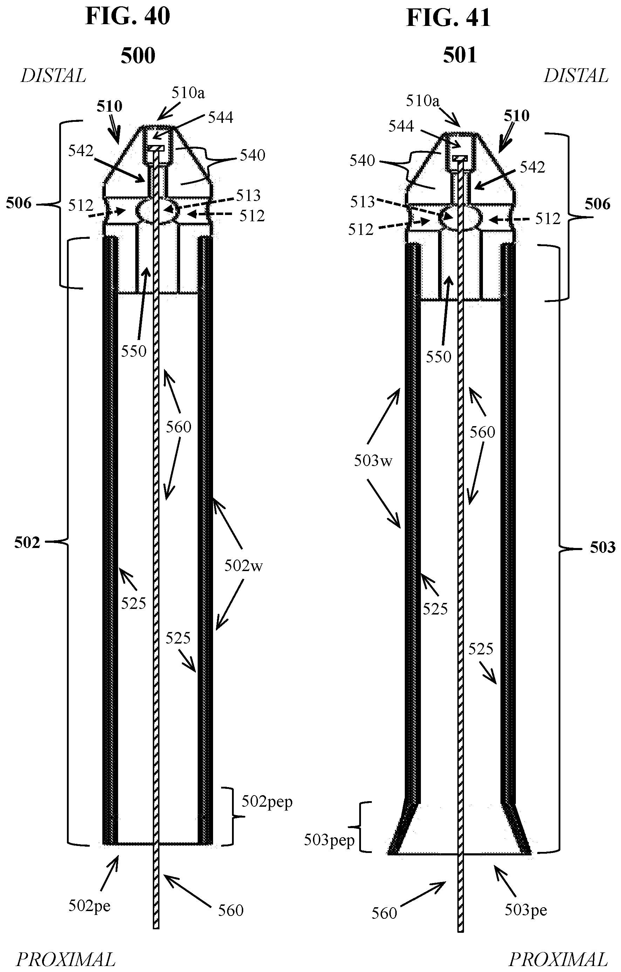

FIGs. 40 and 41 are schematic cross-sectional side views of exemplary embodiments of the sheath, having a *cylindrical* proximal end portion [FIG. 40], and a *conical* proximal end portion [FIG. 41], respectively, highlighting the sheath flexible control wire anchored and fixedly connected to inside of the sheath distal end portion, in accordance with some embodiments of the invention;

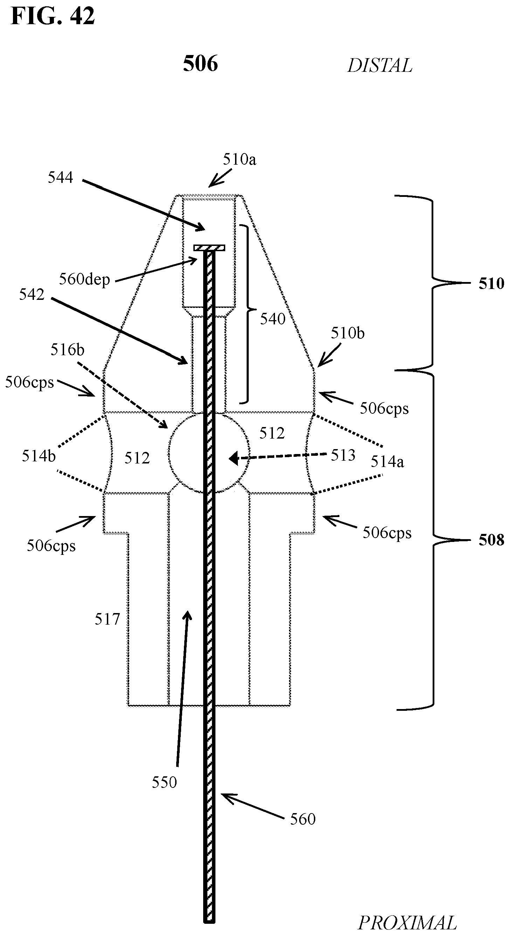

FIG. 42 is a schematic close-up cross-sectional side view of an exemplary embodiment of the sheath distal end portion, highlighting anchoring and fixed connection therein of the sheath flexible control wire, in accordance with some embodiments of the invention;

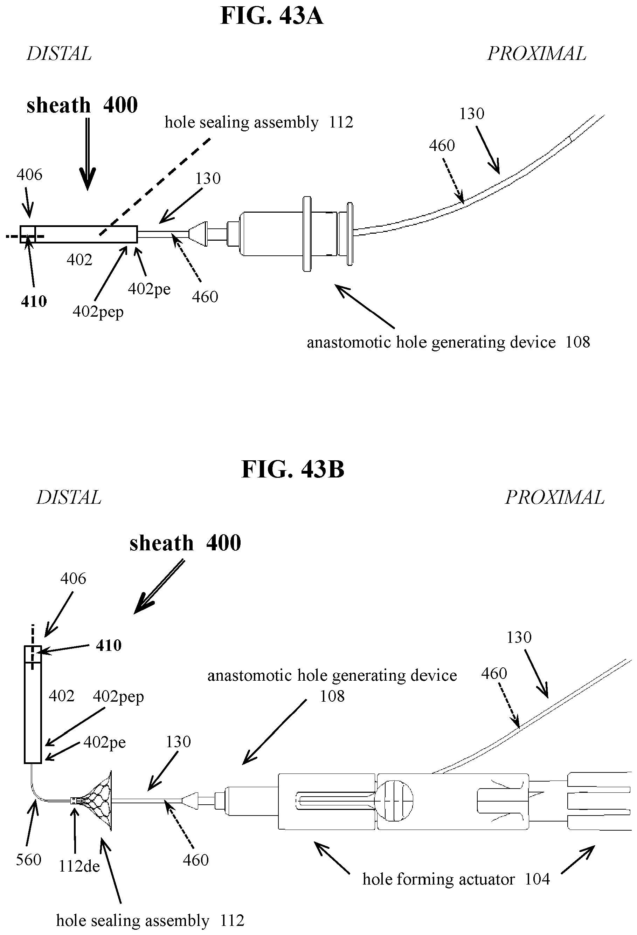

FIGs. 43A and 43B are schematic diagrams of exemplary embodiments of implementing the sheath 400 [including a *single* exemplary intra-sheath fluid (blood, water, air) flow passageway (tunnel) in the sheath distal end portion, and a *cylindrical* proximal end portion], with an exemplary hole sealing assembly, and an exemplary anastomotic hole generating device, in accordance with some embodiments of the invention;

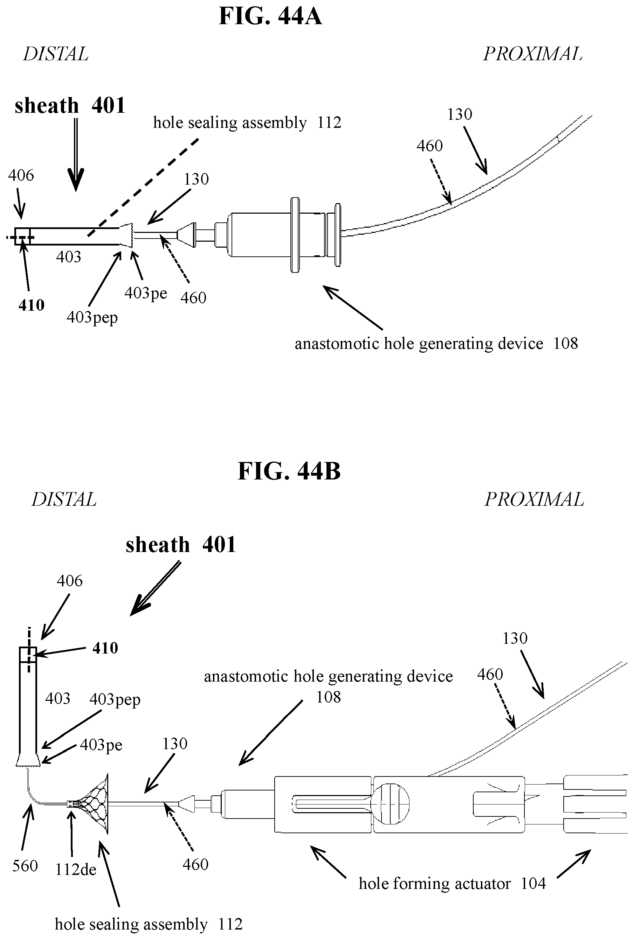

FIGs. 44A and 44B are schematic diagrams of exemplary embodiments of implementing the sheath 401 [including a *single* exemplary intra-sheath fluid (blood, water, air) flow passageway (tunnel) in the sheath distal end portion, and a *conical* proximal end portion], with an exemplary hole sealing assembly, and an exemplary anastomotic hole generating device, in accordance with some embodiments of the invention;

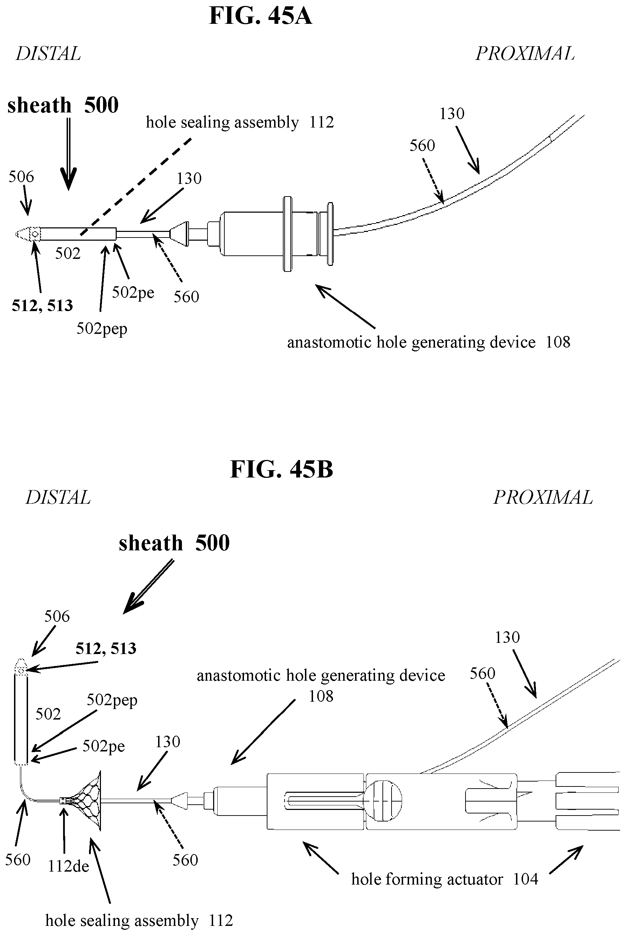

FIGs. 45A and 45B are schematic diagrams of exemplary embodiments of implementing the sheath 500 [including a *plurality* of exemplary intra-sheath fluid (blood, water, air) flow passageways (tunnels) in the sheath distal end portion, and a *cylindrical* proximal end portion], with an exemplary hole sealing assembly, and an exemplary anastomotic hole generating device, in accordance with some embodiments of the invention; and

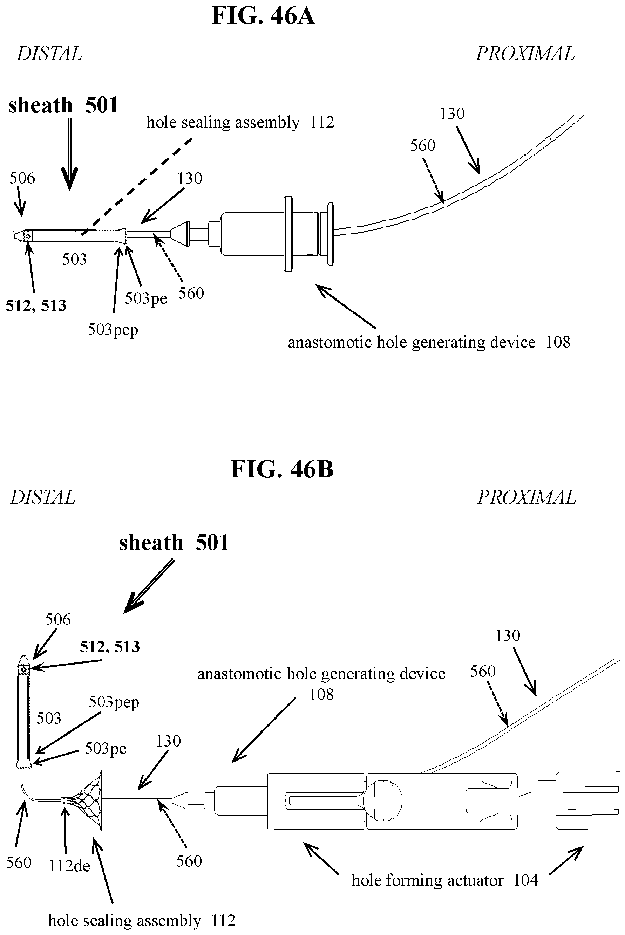

FIGs. 46A and 46B are schematic diagrams of exemplary embodiments of implementing the sheath 501 [including a *plurality* of exemplary intra-sheath fluid (blood, water, air) flow passageways (tunnels) in the sheath distal end portion, and a *conical* proximal end portion], with an exemplary hole sealing assembly, and an exemplary anastomotic hole generating device, in accordance with some embodiments of the invention.

### DESCRIPTION OF SPECIFIC EMBODIMENTS OF THE INVENTION

The present invention, in some embodiments thereof, relates to a sheath for use in performing surgical vascular anastomotic procedures, and applications thereof. Some embodiments of the present invention are particularly applicable for use in 'clampless' types of (end-to-side) surgical vascular anastomotic procedures, for performing coronary artery bypass grafting (CABG).

In exemplary embodiments, the sheath includes a sheath main portion configured as a tubular member and having a sheath main portion distal end and a sheath main portion proximal end, wherein the sheath main portion proximal end is open. The sheath further includes a sheath distal end portion, continuous with the sheath main portion distal end, and including at least one intra-sheath fluid flow passageway, whereby each one of the at least one intra-sheath fluid flow passageway extends and passes entirely through the inside of the sheath distal end portion, via two differently located parts of the sheath distal end portion. Each intra-sheath fluid (blood, water, air) flow passageway (tunnel) inside of the sheath distal end portion is configured (shaped and sized) so as to facilitate *continuous* flow of blood vessel lumen fluids (blood, water, air) through the overall sheath, especially, flow of blood vessel lumen fluids that enter through the sheath (open) proximal end and exit through the sheath (open) distal end portion.

In exemplary embodiments, the sheath includes a *single* intra-sheath fluid (blood, water, air) flow passageway (tunnel) in the sheath distal end portion, or includes *a plurality* of exemplary intra-sheath fluid (blood, water, air) flow passageways (tunnels) in the sheath distal end portion. In such exemplary embodiments, the sheath has either a *cylindrical* (cylindrically shaped) proximal end portion or a *conical* (conically shaped) proximal end portion. In exemplary embodiments, the *conical* proximal end portion may better facilitate pulling of the sheath back onto an activated, self-expanded hole sealing assembly or element, so as to return the hole sealing assembly or element to a non-activated, collapsed configuration, prior to removal of the sheath (with the collapsed hole sealing assembly or element therein) from the blood vessel, and ultimately, from a subject undergoing an anastomotic procedure.

In exemplary embodiments, the sheath distal end portion also includes an intra-sheath control wire anchoring and fixing pocket (cavity), and a sheath control wire passageway (tunnel). In exemplary embodiments, one of the intra-sheath fluid flow passageways (tunnels) is also structured and functional as a combination of: (i) the intra-sheath control wire anchoring and fixing pocket (cavity) and (ii) the sheath control wire passageway (tunnel), inside the sheath distal end portion. In exemplary embodiments, the sheath also includes a sheath flexible control wire, extending along and through the inside of the sheath, and being anchored and fixedly connected to the inside of the sheath distal end portion, via the intra-sheath control wire anchoring and fixing pocket (cavity).

Implementation of the present invention attempts to address, and overcome, at least some of the various problems associated with surgical vascular anastomotic procedures, particularly, those procedures which are employed in coronary artery bypass grafting (CABG). For example, the present invention may be implemented, without need for using clamps, in anastomotic procedures for *atraumatically* generating a peripherally sealed anastomotic hole in a blood vessel inner wall, thereby preventing or minimizing peripheral blood leakage around the anastomotic hole, while maintaining blood flow in the host blood vessel. Such implementation provides safer surgical conditions, and precludes or significantly reduces need for heart surgery teams to perform complex operational activities for peripherally sealing anastomotic holes in blood vessels, in addition to and while performing anastomotic procedures.

In same applicant/assignee PCT Int'l. Pub. No. WO 2018/203237 A1 (Nov. 11, 2018), disclosed are apparatuses and methods for use in surgical vascular anastomotic procedures. Therein, an exemplary apparatus includes a blood vessel inner wall (BVIW) sealing and hole forming device, and a hole forming actuator. The BVIW sealing and hole forming device includes a hole sealing device, and an anastomotic hole generating device. The hole sealing device includes a sheath, a hole sealing assembly, a manual hole sealing controller assembly, and a sheath flexible control wire enclosed within a flexible tube.

As disclosed therein, in exemplary embodiments, the sheath is configured: (i) to externally cover, closely fit over, fully enclose, and a hold the hole sealing assembly in a non-activated, collapsed configuration; and (ii) to *atraumatically* entirely (with the collapsed hole sealing assembly therein) enter into a blood vessel lumen (for example, of an aorta), by passing through a relatively very small hole (having an exemplary diameter of about 0.8 - 1.0 mm) previously made, for example, by a medical practitioner using a needle or syringe, through a blood vessel wall and along a blood vessel inner wall segment thereof. As further disclosed therein, in exemplary embodiments, the sheath is configured as a tubular member having a main portion (i.e., a shaft) whose proximal end is opened, and a conical (i.e., cone or conical shaped) distal end portion whose distal end (i.e., tapered point or apex) is closed. In such exemplary embodiments, the sheath conical distal end has a maximum outer diameter that is less than the outer diameter of the sheath main portion, and also less than the diameter of the very small hole (previously) made through the blood vessel wall.

During actual (*in-vivo*) implementation of such exemplary embodiments of the sheath (having a conical distal end portion whose distal end (tapered point or apex) is closed), the inventors observed that when the sheath is pulled back onto and over the hole sealing assembly (so as to return the hole sealing assembly to its non-activated, collapsed configuration), entering of the hole sealing assembly back into the sheath displaces fluids (especially, blood, and, also water or/and air) *previously trapped* in the blood vessel lumen, which then flow out from inside the sheath proximal end, accompanied by some difficulty in fully returning the hole sealing assembly back into the sheath. The inventors concluded that in such scenarios, following insertion of the sheath (with the collapsed hole sealing assembly therein) into the blood vessel lumen, and after removal of the sheath from the collapsed hole sealing assembly, blood vessel lumen fluids (blood, water, air) become trapped inside the sheath. Such trapping of blood vessel lumen fluids inside the sheath causes an increase in pressure inside the sheath, which makes it harder to subsequently pull the sheath back onto and over the expanded hole sealing assembly, in order to return the hole sealing assembly to its non-activated, collapsed configuration inside the sheath.

The inventors also observed, in some instances, that blood accumulates inside the sheath during anastomosis construction (e.g., requiring about ten minutes), to an extent of forming a thrombus that takes up space inside the sheath, which, in turn, makes it very difficult, if not impossible, to fully close and return the hole sealing assembly back into the sheath (since the sheath is filled with the thrombus, thereby leaving insufficient or no space in the sheath for fully containing the hole sealing assembly).

By way of addressing, and overcoming, the above described limitation associated with such exemplary embodiments of the sheath (i.e., having aa conical distal end portion whose distal end (tapered point or apex) is closed), the inventors developed new exemplary embodiments of a sheath including a sheath main portion configured as a tubular member and having a sheath main portion distal end and a sheath main portion proximal end, where the sheath main portion proximal end is open. In such exemplary embodiments, the sheath also includes a sheath distal end portion, continuous with the sheath main portion distal end, and having at least one intra-sheath fluid (blood, water, air) flow passageway (tunnel), whereby each one of the at least one intra-sheath fluid flow passageway (tunnel) extends and passes entirely through the inside of the sheath distal end portion, via two differently located parts of the sheath distal end portion.

According to such new exemplary embodiments of a sheath, each intra-sheath fluid (blood, water, air) flow passageway (tunnel) inside of the sheath distal end portion is configured (shaped and sized) so as to facilitate *continuous* flow of blood vessel lumen fluids (blood, water, air) through the overall sheath, especially, flow of blood vessel lumen fluids that enter through the sheath (open) proximal end and exit through the sheath (open) distal end portion. In such new exemplary embodiments of a sheath, each intra-sheath fluid (blood, water, air) flow passageway (tunnel) has a diameter large enough to readily facilitate continuous flow of blood vessel lumen fluids through the overall sheath, thereby preventing accumulation of blood and possible thrombus formation inside the sheath. The inventors observed that, with such new exemplary embodiments of a sheath, even in scenarios including (temporary) accumulation of blood and thrombus formation inside the sheath, when the sheath is pulled back onto and over the hole sealing assembly (so as to return the hole sealing assembly to its non-activated, collapsed configuration), entering of the hole sealing assembly back into the sheath readily pushes and displaces the accumulated blood and thrombus out from inside the sheath, through the one or more intra-sheath fluid flow passageways (tunnels), and eventually through and out of the sheath distal end portion.

As further disclosed in same applicant/assignee PCT Int'l. Pub. No. WO 2018/203237 A1, in exemplary embodiments, the inside of the sheath distal end portion, for example, at the distal end thereof, is fixedly connected to the distal end of a sheath flexible control wire that controls motion and positioning of the sheath and conversion (activation) of the hole sealing assembly from its non-activated, collapsed configuration to its activated, self-expanded configuration, and vice versa. For the objective of improving anchoring and fixing of the distal end portion of the flexible control wire to the inside of the sheath distal end portion, the inventors also developed new exemplary embodiments of a sheath wherein the inside of the sheath distal end portion is configured with an intra-sheath control wire anchoring and fixing pocket (or cavity). The intra-sheath control wire anchoring and fixing pocket (cavity) is configured (shaped and sized) so as to prevent, or at least minimize, possible latitudinal or/and (proximal-distal) longitudinal movements of the sheath control wire distal end portion that could lead to displacement thereof, or even exiting of the sheath control wire from inside the sheath distal end portion.

For purposes of further understanding exemplary embodiments of the present invention, in the following illustrative description thereof, reference is made to the figures. Throughout the following description and accompanying drawings, same reference numbers refer to same structures, components, elements, or features, of the herein disclosed invention. Additionally, throughout the description the standard terms *"proximal"* and *"distal"* are used for indicating relative locations, positions, and directions of structures, components, elements, or features, of the herein disclosed invention. For clarity and consistency, these same terms also appear in the drawings. Exemplary materials of construction and size dimensions of components, elements, and structural features of the herein disclosed invention are separately provided near the end of the Description.

It is to be understood that the invention is not necessarily limited in its application to particular details of construction or/and arrangement of exemplary apparatus or/and device components, or to any particular sequential ordering of exemplary method steps or procedures, set forth in the following illustrative description. Additionally, the invention is not necessarily limited in its application for use in surgical vascular anastomotic procedures. The invention is capable of other exemplary embodiments, and of being practiced or carried out in various ways, in various medical applications.

An aspect of the present invention is that of a sheath for use in performing surgical vascular anastomotic procedures. In exemplary embodiments, the sheath includes a sheath main portion configured as a tubular member and having a sheath main portion distal end and a sheath main portion proximal end, where the sheath main portion proximal end is open. In such exemplary embodiments, the sheath also includes a sheath distal end portion, continuous with the sheath main portion distal end, and having at least one intra-sheath fluid (blood, water, air) flow passageway (tunnel), whereby each one of the at least one intra-sheath fluid flow passageway (tunnel) extends and passes entirely through the inside of the sheath distal end portion, via two differently located parts of the sheath distal end portion. In exemplary embodiments, the sheath includes, for example, two or more intra-sheath fluid (blood, water, air) flow passageways (tunnels).

### Exemplary embodiments of a sheath with a single exemplary intra-sheath fluid (blood, water, air) flow passageway (tunnel) in the sheath distal end portion

FIGs. 1 and 2 are schematic perspective and side views, respectively, of an exemplary embodiment of the sheath [indicated as, and referred to by, reference number 400], having a *cylindrical* (cylindrically shaped) proximal end portion, highlighting external appearance of a *single* exemplary intra-sheath fluid (blood, water, air) flow passageway (tunnel) in the sheath distal end portion. FIGs. 3 and 4 are schematic perspective and side views, respectively, of another exemplary embodiment of the sheath [indicated as, and referred to by, reference number 401], having a *conical* (conically shaped) proximal end portion, highlighting external appearance of a *single* exemplary intra-sheath fluid (blood, water, air) flow passageway (tunnel) in the sheath distal end portion.

FIGs. 5 - 19 provide additional illustrations of exemplary embodiments of the sheaths 400 and 401, and of the various structural and functional (operational) features and characteristics thereof. FIGs. 43A - 43B, and 44A - 44B, illustrate exemplary application of the sheaths 400 and 401.

Although FIGs. 1 - 19 illustrate exemplary sheaths 400 and 401 including a *single* exemplary intra-sheath fluid (blood, water, air) flow passageway (tunnel) 410 in the sheath distal end portion 406, in a non-limiting manner, exemplary sheaths 400 or 401 may also have *a plurality* of (two or more) exemplary intra-sheath fluid (blood, water, air) flow passageways (tunnels) in the sheath distal end portion 406, whereby each intra-sheath fluid flow passageway (tunnel) extends and passes entirely through the inside of the sheath distal end portion 406, via two differently located parts of the sheath distal end portion 406. For example, as illustratively described further hereinbelow, FIGs. 20 - 42 illustrate exemplary sheaths 500 and 501 including *a plurality* of exemplary intra-sheath fluid (blood, water, air) flow passageways (tunnels) in the sheath distal end portion 506.

Exemplary sheath 401 shown in FIGs. 3 and 4 has the same structural features and characteristics as exemplary sheath 400 shown in FIGs. 1 and 2, except for the particular geometrical shape (and size dimensions thereof) of the sheath proximal end portion. Namely, in FIGs. 1 and 2, exemplary sheath 400, and sheath main portion 402 thereof, have a *cylindrical* proximal end portion 402pep, whereas, in FIGs. 3 and 4, exemplary sheath 401, and sheath main portion 403, have a *conical* proximal end portion 403pep, with correspondingly different size dimensions for each respective proximal end portion.

As shown in FIGs. 1 and 2, in exemplary embodiments, sheath 400 includes: a sheath main portion 402 configured as a tubular member (e.g., a hollow shaft) and having a sheath main portion distal end 402de and a sheath main portion proximal end 402pe, where the sheath main portion proximal end 402pe is open. Sheath 400 also includes a sheath distal end portion 406, continuous with the sheath main portion distal end 402de, and having at least one (for example, a single) exemplary intra-sheath fluid (blood, water, air) flow passageway 410 [for example, as indicated in FIGs. 1 and 2 by the dashed line 410] that extends and passes entirely through the inside of the sheath distal end portion 406, via two differently located parts, for example, parts 406a and 406b [for example, referenced in the drawings, by dotted lines], of the sheath distal end portion 406.

As shown in FIGs. 3 and 4, in exemplary embodiments, sheath 401 includes: a sheath main portion 403 configured as a tubular member (e.g., a hollow shaft) and having a sheath main portion distal end 403de and a sheath main portion proximal end 403pe, where the sheath main portion proximal end 403pe is open. Sheath 401 also includes a sheath distal end portion 406, continuous with the sheath main portion distal end 403de, and having a single exemplary intra-sheath fluid (blood, water, air) flow passageway 410 [for example, as indicated in FIGs. 3 and 4 by the dashed line 410] that extends and passes entirely through the inside of the sheath distal end portion 406, via two differently located parts, for example, parts 406a and 406b, of the sheath distal end portion 406.

In exemplary sheaths 400 and 401, the sheath main portion (tubular member) 402 and 403, respectively, has a wall 402w and 403w, respectively, that (proximally-distally) extends along the entirety of the sheath main portion 402 and 403, respectively. In exemplary sheath 400, the proximal end portion 402pep is cylindrical, for example, having the same cylindrical shape and size dimensions as that of the sheath main portion (tubular member) 402. In exemplary sheath 401, the proximal end portion 403pep is conical, thereby, having a different geometrical shape relative to the remainder of the cylindrical sheath main portion (tubular member) 403. In exemplary sheath 401, the proximal end portion 403pep has an apex 403a and a base 403b, whereby the diameter of the base 403b is larger than the diameter of the apex 403a. In some applications of the invention (for example, as shown in FIGs. 44A - 44B), use of exemplary sheath 401 having the *conical* proximal end portion 403pep, with the diameter of the base 403b being larger than the diameter of the apex 403a, may better facilitate pulling of the sheath 401 back onto an activated, self-expanded hole sealing assembly or element, so as to return the hole sealing assembly or element to a non-activated, collapsed configuration, prior to removal of the sheath 401 (with the collapsed hole sealing assembly or element therein) from a blood vessel, and ultimately, from a subject undergoing an anastomotic procedure.

In exemplary embodiments, the sheath distal end portion 406 is configured as a cylindrical member having a distal end 406de, and a proximal end 406pe (for example, as shown in FIGs. 5, 6, 10, and 12 - 14). Each of the distal end 406de and the proximal end 406pe includes an opening (e.g., a hole) corresponding to the (distal and proximal) openings of the single exemplary intra-sheath fluid (blood, water, air) flow passageway 410 that extends and passes entirely through the inside of the sheath distal end portion 406, via the two differently (for example, oppositely or diametrically opposed) located parts 406a and 406b, respectively, of the sheath distal end portion 406.

In exemplary embodiments, the sheath main portion (shaft) 402 or 403 and the sheath distal end portion 406 are configured as a single, integral type of monolithic structure, wherein the sheath main portion (shaft) 402 or 403 and the sheath distal end portion 506 are integrally formed as a single continuous structure. In alternative exemplary embodiments, the sheath main portion (shaft) 402 or 403 and the sheath distal end portion 406 are configured as two individual, operably connectable (attachable), structures (components), for example, most noticeable in FIGs. 5, 6, 10, and 12.

FIGs. 5 and 6 are schematic exploded side views of exemplary embodiments of the sheath, having a *cylindrical* proximal end portion 402pep [FIG. 5, sheath 400], and a *conical* proximal end portion 403pep [FIG. 6], respectively, highlighting the sheath main portion (tubular member) 402 or 403 and the sheath distal end portion 406 configured as two individual, operably connectable structures (components). In such exemplary embodiments, for example, the cylindrical sheath distal end portion 406 is configured with a proximal end portion 406pep that closely (snugly) fits into the tubular distal end portion 402dep of the sheath main portion 402 or 403. For example, whereby the proximal end portion 406pep has an outer diameter slightly less than the inner diameter of the distal end portion 402dep of the sheath main portion 402 or 403.

In exemplary embodiments, the sheath main portion (shaft) 402 or 403 external or outer surface is coated with a coating material. FIGs. 7 and 8 are schematic side views of exemplary embodiments of the sheath main portion (shaft) 402 or 403 having a *cylindrical* proximal end portion 402pep [FIG. 7], or a *conical* proximal end portion 403pep [FIG. 8], respectively, highlighting a coating, for example, coating 420, upon the external surface of the respective sheath main portion (tubular member) 402 or 403.

In exemplary embodiments, the sheath main portion (shaft) 402 or 403 internal or inner surface is coated (lined) with a coating (lining) material, for example, coating (lining) material 425, as shown in FIGs. 9 - 12, 17, and 18. In exemplary embodiments, the coating material 425 is a friction reducing type of coating material. In such exemplary embodiments, use of a friction reducing type of internal or inner surface coating material assists with facilitating pulling of the sheath 400 or 401 back onto an activated, self-expanded hole sealing assembly or element, so as to return the hole sealing assembly or element to a non-activated, collapsed configuration, prior to removal of the sheath (with the collapsed hole sealing assembly or element therein) from the blood vessel, and ultimately, from a subject undergoing an anastomotic procedure.

### Intra-sheath fluid (blood, water, air) flow passageway (tunnel)

As shown in the drawings (for example, FIGs. 1 - 6, and 9 - 12) in exemplary sheath 400 or 401, the sheath distal end portion 406 is continuous with the sheath main portion distal end 402de or 403de, and includes a single exemplary intra-sheath fluid (blood, water, air) flow passageway (tunnel) 410 that extends and passes entirely through the inside of the sheath distal end portion 406, via two differently located parts, for example, parts 406a and 406b of the sheath distal end portion 406. In such exemplary embodiments, the exemplary intra-sheath fluid (blood, water, air) flow passageway (tunnel) 410 extends and passes entirely through the middle or center (proximal-distal) longitudinal axis inside of the sheath distal end portion 406, via the two oppositely (diametrically opposed), and centrally, located parts 406a and 406b of the sheath distal end portion 406.

Extension and passage of the exemplary intra-sheath fluid flow passageway (tunnel) 410 are not limited to being entirely along and through the middle or center (proximal-distal) longitudinal axis inside of the sheath distal end portion 406, via the two oppositely (diametrically opposed), and centrally, located parts 406a and 406b of the sheath distal end portion 406. In alternative exemplary embodiments, the exemplary intra-sheath fluid (blood, water, air) flow passageway 410 extends and passes entirely through a non-middle or off-center (proximal-distal) longitudinal axis inside of the sheath distal end portion 406, via the two oppositely (diametrically opposed), and non-centrally, located parts 406a and 406b of the sheath distal end portion 406.

Several additional alternative exemplary embodiments are possible for extension and passage of one or more of the at least one intra-sheath fluid flow passageway (tunnel) entirely through the inside of the sheath distal end portion 406, via a corresponding number of pairs of two differently located parts of the sheath distal end portion 406. For example, exemplary sheath 400 or 401 may be designed and constructed such that sheath distal end portion 406 includes a plurality of two or more intra-sheath fluid flow passageways (tunnels), for example, a plurality of two or more of the single intra-sheath fluid flow passageways (tunnels) 410, whereby each one of the plurality of two or more intra-sheath fluid flow passageways (tunnels) 410 extends and passes entirely through the inside of the sheath distal end portion 406, via a corresponding number of pairs of two differently located parts of the sheath distal end portion 406. In additional alternative exemplary embodiments, at least one intra-sheath fluid flow passageway (tunnel) may radially (orthogonally or non-orthogonally to the proximal - distal directions) extend and pass entirely through the inside of the sheath distal end portion 406, via two differently located parts of the sheath distal end portion 406.

According to such exemplary embodiments of the sheath 400 or 401, the intra-sheath fluid (blood, water, air) flow passageway (tunnel) 410 inside of the sheath distal end portion 406 is configured (shaped and sized) so as to facilitate *continuous* flow of blood vessel lumen fluids (blood, water, air) through the overall sheath 400 or 401, especially, flow of blood vessel lumen fluids that enter through the sheath (open) proximal end 402pe or 403pe, respectively, and exit through the sheath (open) distal end portion 406. In such exemplary embodiments, the intra-sheath fluid (blood, water, air) flow passageway (tunnel) 410 has a diameter large enough to readily facilitate continuous flow of blood vessel lumen fluids through the overall sheath 400 or 401, thereby preventing accumulation of blood and possible thrombus formation inside the sheath. Even in scenarios including (temporary) accumulation of blood and thrombus formation inside the sheath 400 or 401, when the sheath is pulled back onto and over a hole sealing assembly (so as to return the hole sealing assembly to its non-activated, collapsed configuration), entering of the hole sealing assembly back into the sheath readily pushes and displaces the accumulated blood and thrombus out from inside the sheath, through the intra-sheath fluid flow passageway (tunnel) 410, and eventually through and out of the sheath distal end portion 406.

### Intra-sheath control wire anchoring and fixing pocket (cavity), and sheath control wire passageway (tunnel)

As explained hereinabove, for the objective of improving anchoring and fixing of the distal end portion of a sheath flexible control wire to the inside of the sheath distal end portion, the inventors also developed new exemplary embodiments of a sheath wherein the inside of the sheath distal end portion is also configured with an intra-sheath control wire anchoring and fixing pocket (or cavity). The intra-sheath control wire anchoring and fixing pocket (cavity) is configured (shaped and sized) so as to prevent, or at least minimize, possible latitudinal or/and (proximal-distal) longitudinal movement of a sheath control wire distal end portion that could lead to displacement thereof, or even exiting of the sheath control wire from inside the sheath distal end portion.

FIGs. 9 and 10 are schematic cross-sectional, and exploded cross-sectional, side views, respectively, of exemplary embodiments of the sheath 400, having a *cylindrical* proximal end portion 402pep, highlighting internal appearance and configuration of the *single* exemplary intra-sheath fluid (blood, water, air) flow passageway (tunnel) 410 that is also structured and functional as a combination of an exemplary intra-sheath control wire anchoring and fixing pocket (cavity) 440 (442 + 444) and an exemplary sheath control wire passageway (tunnel) 450, inside the sheath distal end portion 406.

FIGs. 11 and 12 are schematic cross-sectional, and exploded cross-sectional, side views, respectively, of exemplary embodiments of the sheath 401, having a *conical* proximal end portion 403pep, highlighting internal appearance and configuration of the *single* exemplary intra-sheath fluid (blood, water, air) flow passageway (tunnel) 410 that is also structured and functional as a combination of an exemplary intra-sheath control wire anchoring and fixing pocket (cavity) 440 (442 + 444) and an exemplary sheath control wire passageway (tunnel) 450, inside the sheath distal end portion 406.

FIGs. 13 and 14 are a schematic close-up perspective view, and a schematic close-up cross-sectional side view, respectively, of an exemplary embodiment of the sheath distal end portion 406 of exemplary sheaths 400 and 401.

FIGs. 15 and 16 are schematic perspective views of exemplary embodiments of the sheaths 400 and 401, having a *cylindrical* proximal end portion [FIG. 15], and a *conical* proximal end portion [FIG. 16], respectively, highlighting inclusion of an exemplary sheath control wire 460 extending along and through the inside of the sheath.

FIGs. 17 and 18 are schematic cross-sectional side views of exemplary embodiments of the sheaths 400 and 401, having a *cylindrical* proximal end portion [FIG. 17], and a *conical* proximal end portion [FIG. 18], respectively, highlighting the sheath control wire 460 anchored and fixedly connected to the inside of the sheath distal end portion 406. FIG. 19 is a schematic close-up cross-sectional side view of an exemplary embodiment of the sheath distal end portion 406, highlighting anchoring and fixed connection therein of the sheath control wire 460.

In exemplary embodiments, the intra-sheath control wire anchoring and fixing pocket (cavity) 440 (442 + 444) is tubular and includes two portions, namely, a first portion 442 and a second portion 444. In exemplary embodiments, the sheath control wire 460 has a distal end portion 460dep (for example, as shown in FIG. 19) that is located in the second portion 444 of the intra-sheath control wire anchoring and fixing pocket (cavity) 440. In exemplary embodiments, the intra-sheath control wire anchoring and fixing pocket (cavity) 440, and the sheath control wire 460, are configured (shaped and sized) relative to each other, so as to prevent, or at least minimize, possible latitudinal or/and (proximal-distal) longitudinal movements of the sheath control wire distal end portion 460dep that could lead to displacement thereof, or even exiting of the sheath control wire 460 from inside the sheath distal end portion 406.

In exemplary embodiments, the inner diameter of the first portion 442 is less than the inner diameter of the second portion 444. In exemplary embodiments, the sheath control wire distal end portion 460dep is positioned, anchored, and fixedly connected (attached) (e.g., using an adhesive or glue, or similar type of fixing material) to the inside of the second portion 444 (being the larger portion) of the intra-sheath control wire anchoring and fixing pocket (cavity) 440. Following anchoring and fixing of the sheath control wire distal end portion 460dep to the inside of the sheath distal end portion 406, such multi-portion (multi-stage) configuration of the intra-sheath control wire anchoring and fixing pocket (cavity) 440 restricts latitudinal and (proximal-distal) longitudinal movements of the sheath control wire 460 therein. Such is particularly relevant in exemplary embodiments wherein the outer diameter of the sheath control wire 460 is significantly less than the inner diameter of the first portion 442 (and therefore, even further less than the inner diameter of the second portion 444), whereby the possibility exists for the sheath control wire 460 to undergo latitudinal or/and (proximal-distal) longitudinal shifting type of movements inside the sheath distal end portion 406.

In exemplary embodiments, the inside of the proximal portion of the sheath distal end portion 406 is also configured with a sheath control wire passageway (tunnel), for example, sheath control wire passageway (tunnel) 450 (for example, as shown in FIGs. 9 - 12, 14, and 17 - 19). Exemplary sheath control wire passageway (tunnel) 450 is configured (shaped and sized) so as to provide a passageway (tunnel) within and along which a sheath control wire (for example, sheath control wire 460) is held, controlled, and guided, for example, by manual operation of a hole sealing device (for example, hole sealing device 106 disclosed in same applicant/assignee PCT Int'l. Pub. No. WO 2018/203237 A1). In exemplary embodiments, the sheath control wire passageway (tunnel) 450 has an inner diameter that is larger than the inner diameter of each of the first and second portions 442 and 444, respectively, of the intra-sheath control wire anchoring and fixing pocket (cavity) 440.

In exemplary embodiments of sheaths 400 and 401, the single exemplary intra-sheath fluid (blood, water, air) flow passageway (tunnel) 410 is also structured and functional as a combination of the exemplary intra-sheath control wire anchoring and fixing pocket (cavity) 440 (442 + 444) and the exemplary sheath control wire passageway (tunnel) 450.

### Exemplary embodiments of a sheath with a plurality of exemplary intra-sheath fluid (blood, water, air) flow passageways (tunnels) in the sheath distal end portion

FIGs. 20 and 21 are schematic perspective and side views, respectively, of an exemplary embodiment of the sheath [indicated as, and referred to by, reference number 500], having a *cylindrical* proximal end portion, highlighting external appearance of a *plurality* of exemplary intra-sheath fluid (blood, water, air) flow passageways (tunnels) in the sheath distal end portion. FIGs. 22 and 23 are schematic perspective and side views, respectively, of another exemplary embodiment of the sheath [indicated as, and referred to by, reference number 501], having a *conical* proximal end portion, highlighting external appearance of a *plurality* of exemplary intra-sheath fluid (blood, water, air) flow passageways (tunnels) in the sheath distal end portion.

FIGs. 24 - 42 provide additional illustrations of exemplary embodiments of the sheaths 500 and 501, and of the various structural and functional (operational) features and characteristics thereof. FIGs. 45A - 45B, and 46A - 46B, illustrate exemplary application of the sheaths 500 and 501.

Exemplary sheath 500 (and features thereof) shown in FIG. 21 corresponds to a 45° counter-clockwise rotation (around a longitudinal axis orthogonal to the proximal - distal directions) of that shown in FIG. 20. Similarly, exemplary sheath 501 (and features thereof) shown in FIG. 23 corresponds to a 45° counter-clockwise rotation (around a longitudinal axis orthogonal to the proximal - distal directions) of that shown in FIG. 22.

Exemplary sheath 501 shown in FIGs. 22 and 23 has the same structural features and characteristics as exemplary sheath 500 shown in FIGs. 20 and 21, except for the particular geometrical shape (and size dimensions thereof) of the sheath proximal end portion. Namely, in FIGs. 20 and 21, exemplary sheath 500, and sheath main portion 502 thereof, have a *cylindrical* (cylindrically shaped) proximal end portion 502pep, whereas, in FIGs. 22 and 23, exemplary sheath 501, and sheath main portion 503, have a *conical* (conically shaped) proximal end portion 503pep, with correspondingly different size dimensions for each respective proximal end portion.

As shown in FIGs. 21 and 22, in exemplary embodiments, sheath 500 includes: a sheath main portion 502 configured as a tubular member (e.g., a hollow shaft) and having a sheath main portion distal end 502de and a sheath main portion proximal end 502pe, where the sheath main portion proximal end 502pe is open. Sheath 500 also includes a sheath distal end portion 506, continuous with the sheath main portion distal end 502de, and having at least one exemplary intra-sheath fluid (blood, water, air) flow passageway that extends and passes entirely through the inside of the sheath distal end portion 506, via two differently located parts of the sheath distal end portion 506.

For example, FIGs. 20 and 21 show sheath distal end portion 506 including two intra-sheath fluid (blood, water, air) flow passageways (tunnels) 512 and 513 (indicated by the dashed line arrows leading into or entering the cylindrical base section 508 of the sheath distal end portion 506), whereby each one of the intra-sheath fluid flow passageways (tunnels) 512 and 513 extends and passes entirely through the inside of the sheath distal end portion 506, via two differently located parts of the sheath distal end portion 506.

In exemplary embodiments, each one of the intra-sheath fluid flow passageways (tunnels) 512 and 513 radially (orthogonally to the proximal - distal directions) extends and passes entirely through the inside of the sheath distal end portion 506, via a pair of two oppositely located (diametrically opposed) parts of a circumferential periphery surface of the sheath distal end portion 506 (herein, referred to, and indicated in the drawings, as 506cps). More specifically, in exemplary sheath 500, intra-sheath fluid flow passageway (tunnel) 512 radially (orthogonally to the proximal - distal directions) extends and passes entirely through the inside of the sheath distal end portion 506, via a pair of two oppositely located (diametrically opposed) first and second parts 514a and 514b [for example, referenced in the drawings by dotted lines], respectively, of the circumferential periphery surface 506cps of the sheath distal end portion 506. Similarly, intra-sheath fluid flow passageway (tunnel) 513 radially (orthogonally to the proximal - distal directions) extends and passes entirely through the inside of the sheath distal end portion 506, via a pair of two oppositely located (diametrically opposed) first and second parts 516a and 516b, respectively, of the circumferential periphery surface 506cps of the sheath distal end portion 506. In FIGs. 20 and 21, the second part 516b is present, but, not visible (i.e., being behind, and on the opposite side of, the plane of the displayed page), as indicated in FIG. 21 by the dashed line arrow with reference number 516b.

As shown in FIGs. 22 and 23, in exemplary embodiments, sheath 501 includes: a sheath main portion 503 configured as a tubular member (e.g., a hollow shaft) and having a sheath main portion distal end 503de and a sheath main portion proximal end 503pe, where the sheath main portion proximal end 503pe is open. Sheath 501 also includes a sheath distal end portion 506, continuous with the sheath main portion distal end 503de, and having at least one exemplary intra-sheath fluid (blood, water, air) flow passageway that extends and passes entirely through the inside of the sheath distal end portion 506, via two differently located parts of the sheath distal end portion 506.

For example, FIGs. 22 and 23 show sheath distal end portion 506 including a plurality of two intra-sheath fluid (blood, water, air) flow passageways (tunnels) 512 and 513 (indicated by the dashed line arrows leading into or entering the cylindrical base section 508 of the sheath distal end portion 506), whereby each one of the intra-sheath fluid flow passageways (tunnels) 512 and 513 extends and passes entirely through the inside of the sheath distal end portion 506, via two differently located parts of the sheath distal end portion 506.

In exemplary embodiments, each one of the intra-sheath fluid flow passageways (tunnels) 512 and 513 radially (orthogonally to the proximal - distal directions) extends and passes entirely through the inside of the sheath distal end portion 506, via a pair of two oppositely located (diametrically opposed) parts of the circumferential periphery surface 506cps of the sheath distal end portion 506. More specifically, in exemplary sheath 501, intra-sheath fluid flow passageway (tunnel) 512 radially (orthogonally to the proximal - distal directions) extends and passes entirely through the inside of the sheath distal end portion 506, via a pair of two oppositely located (diametrically opposed) first and second parts 514a and 514b, respectively, of the circumferential periphery surface 506cps of the sheath distal end portion 506. Similarly, intra-sheath fluid flow passageway (tunnel) 513 radially (orthogonally to the proximal - distal directions) extends and passes entirely through the inside of the sheath distal end portion 506, via a pair of two oppositely located (diametrically opposed) first and second parts 516a and 516b, respectively, of the circumferential periphery surface 506cps of the sheath distal end portion 506. In FIGs. 22 and 23, the second part 516b is present, but, not visible (i.e., being behind, and on the opposite side of, the plane of the displayed page), as indicated in FIG. 23 by the dashed line arrow with reference number 516b.

In exemplary sheaths 500 and 501, the sheath main portion (tubular member) 502 and 503, respectively, has a wall 502w and 503w, respectively, that (proximally-distally) extends along the entirety of the sheath main portion 502 and 503, respectively. In exemplary sheath 500, the proximal end portion 502pep is cylindrical, for example, having the same cylindrical shape and size dimensions as that of the sheath main portion (tubular member) 502. In exemplary sheath 501, the proximal end portion 503pep is conical, thereby, having a different geometrical shape relative to the remainder of the cylindrical sheath main portion (tubular member) 503. **In** exemplary sheath 501, the proximal end portion 503pep has an apex 503a and a base 503b, whereby the diameter of the base 503b is larger than the diameter of the apex 503a. **In** some applications of the invention (for example, as shown in FIGs. 46A - 46B), use of exemplary sheath 501 having the *conical* proximal end portion 503pep, with the diameter of the base 503b being larger than the diameter of the apex 503a, may better facilitate pulling of the sheath 501 back onto an activated, self-expanded hole sealing assembly or element, so as to return the hole sealing assembly or element to a non-activated, collapsed configuration, prior to removal of the sheath 501 (with the collapsed hole sealing assembly or element therein) from a blood vessel, and ultimately, from a subject undergoing an anastomotic procedure.

**In** exemplary embodiments, the sheath distal end portion 506 is configured as a member having a cylindrical base section 508 and a conical top section 510 thereupon. The conical top section 510 has an apex 510a and a base 510b, whereby the diameter of the base 510b is larger than the diameter of the apex 510a. **In** exemplary embodiments, in the sheath distal end portion 506, the cylindrical base section 508 includes openings (e.g., holes) corresponding to the four openings of the two intra-sheath fluid (blood, water, air) flow passageways (tunnels) 512 and 513 that radially extend and pass entirely through the inside of the sheath distal end portion 506, via the corresponding two pairs of two oppositely located (diametrically opposed) first and second parts 514a and 514b (for intra-sheath fluid flow passageway 512), and, first and second parts 516a and 516b (for intra-sheath fluid flow passageway 513), respectively, of the circumferential periphery surface 506cps of the sheath distal end portion 506.

**In** exemplary embodiments, the sheath main portion (shaft) 502 or 503 and the sheath distal end portion 506 are configured as a single, integral type of monolithic structure, wherein the sheath main portion (shaft) 502 or 503 and the sheath distal end portion 506 are integrally formed as a single continuous structure. **In** alternative exemplary embodiments, the sheath main portion (shaft) 502 or 503 and the sheath distal end portion 506 are configured as two individual, operably connectable (attachable), structures (components), for example, most noticeable in FIGs. 24, 25, 29, and 31.

FIGs. 24 and 25 are schematic exploded side views of exemplary embodiments of the sheath, having a *cylindrical* proximal end portion [FIG. 24, sheath 500], and a *conical* proximal end portion [FIG. 25, sheath 501], respectively, highlighting the sheath main portion (tubular member) 502 or 503 and the sheath distal end portion (including the cylindrical base 508 and the conical top 510 thereupon) configured as two individual, operably connectable structures (components). In such exemplary embodiments, for example, the sheath distal end portion 506 is configured with a proximal end portion 517 that closely (snugly) fits into the tubular distal end portion 518 of the sheath main portion 502 or 503. For example, whereby the proximal end portion 517 has an outer diameter slightly less than the inner diameter of the distal end portion 518 of the sheath main portion 502 or 503.

In exemplary embodiments, the sheath main portion (shaft) 502 or 503 external or outer surface is coated with a coating material. FIGs. 26 and 27 are schematic side views of exemplary embodiments of the sheath main portion (shaft) 502 or 503, having a *cylindrical* proximal end portion 502pep [FIG. 26], or a *conical* proximal end portion 503pep [FIG. 27], respectively, highlighting a coating, for example, coating 520, upon the external surface of the respective sheath main portion (tubular member) 502 or 503.

In exemplary embodiments, the sheath main portion (shaft) 502 or 503 internal or inner surface is coated (lined) with a coating (lining) material, for example, coating (lining) material 525, as shown in FIGs. 29 - 31, 40, and 41. In exemplary embodiments, the coating material 525 is a friction reducing type of coating material. In such exemplary embodiments, use of a friction reducing type of internal or inner surface coating material assists with facilitating pulling of the sheath 500 or 501 back onto an activated, self-expanded hole sealing assembly or element, so as to return the hole sealing assembly or element to a non-activated, collapsed configuration, prior to removal of the sheath (with the collapsed hole sealing assembly or element therein) from the blood vessel, and ultimately, from a subject undergoing an anastomotic procedure.

### Intra-sheath fluid (blood, water, air) flow passageways (tunnels)

As shown in the drawings (for example, in FIGs. 20 - 25, and 28 - 31), in exemplary sheath 500 or 501, the sheath distal end portion 506 is continuous with the sheath main portion distal end 502de or 503de, and includes a plurality of two intra-sheath fluid (blood, water, air) flow passageways (tunnels) 512 and 513 that radially (orthogonally to the proximal - distal directions) extend and pass entirely through the inside of the sheath distal end portion 506, via the corresponding two pairs of two oppositely located (diametrically opposed) first and second parts 514a and 514b (for intra-sheath fluid flow passageway 512), and, first and second parts 516a and 516b (for intra-sheath fluid flow passageway 513), respectively, of the circumferential periphery surface 506cps of the sheath distal end portion 506.

In such exemplary embodiments, each one of the two intra-sheath fluid (blood, water, air) flow passageways (tunnels) 512 and 513 radially (orthogonally to the proximal - distal directions) extends and passes entirely through the inside of the cylindrical base section 508 of the sheath distal end portion 506, via the corresponding pair of two oppositely located (diametrically opposed) first and second parts 514a and 514b, and, first and second parts 516a and 516b, respectively, of the circumferential periphery surface 506cps of the sheath distal end portion 506.

Extension and passage of either one (or both) of the exemplary intra-sheath fluid flow passageways (tunnels) 512 and 513 are not limited to being radially (orthogonally to the proximal - distal directions) entirely through the inside of the cylindrical base section 508 of the sheath distal end portion 506, via the corresponding pairs of two oppositely located (diametrically opposed) first and second parts 514a and 514b, and, first and second parts 516a and 516b, respectively, of the circumferential periphery surface 506cps of the sheath distal end portion 506.

In alternative exemplary embodiments, either one (or both) of the exemplary intra-sheath fluid (blood, water, air) flow passageways (tunnels) 512 and 513 radially, but, non-orthogonally to the proximal-distal directions, extends and passes entirely through the inside of the sheath distal end portion 506, via two differently located parts of the sheath distal end portion 506. Moreover, in such alternative exemplary embodiments, the two differently located parts of the sheath distal end portion 506 can be the same as, or different from, the two oppositely located (diametrically opposed) first and second parts 514a and 514b, or, first and second parts 516a and 516b, of the circumferential periphery surface 506cps of the sheath distal end portion 506.

For example, in additional exemplary embodiments, at least one intra-sheath fluid (blood, water, air) flow passageway (tunnel) may radially (orthogonally or non-orthogonally to the proximal - distal directions) extend and pass entirely through the inside of the conical top section 510 of the sheath distal end portion 506, via two differently located parts of the circumferential periphery surface of the conical top 510 of the sheath distal end portion 506.

Several additional alternative exemplary embodiments are possible for extension and passage of one or more of the at least one intra-sheath fluid flow passageway (tunnel) entirely through the inside of the sheath distal end portion 506, via a corresponding number of pairs of two differently located parts of the sheath distal end portion 506. For example, exemplary sheath 500 or 501 may be designed and constructed such that sheath distal end portion 506 includes a single intra-sheath fluid flow passageway (tunnel), for example, only one of the two intra-sheath fluid flow passageways (tunnels) 512 or 513, whereby the single intra-sheath fluid flow passageway (tunnel) 512 or 513 extends and passes entirely through the inside of the sheath distal end portion 506, via a single pair of two differently located parts of the sheath distal end portion 506.

According to such exemplary embodiments of the sheath 500 or 501, each on of the intra-sheath fluid (blood, water, air) flow passageways (tunnels) 512 and 513 inside of the sheath distal end portion 506 is configured (shaped and sized) so as to facilitate *continuous* flow of blood vessel lumen fluids (blood, water, air) through the overall sheath 500 or 501, especially, flow of blood vessel lumen fluids that enter through the sheath (open) proximal end 502pe or 503pe, respectively, and exit through the sheath (open) distal end portion 506. In such exemplary embodiments, each intra-sheath fluid (blood, water, air) flow passageway (tunnel) 512 and 513 has a diameter large enough to readily facilitate continuous flow of blood vessel lumen fluids through the overall sheath 500 or 501, thereby preventing accumulation of blood and possible thrombus formation inside the sheath. Even in scenarios including (temporary) accumulation of blood and thrombus formation inside the sheath 500 or 501, when the sheath is pulled back onto and over a hole sealing assembly (so as to return the hole sealing assembly to its non-activated, collapsed configuration), entering of the hole sealing assembly back into the sheath readily pushes and displaces the accumulated blood and thrombus out from inside the sheath, through each intra-sheath fluid flow passageway (tunnel) 512 and 513, and eventually through and out of the sheath distal end portion 506.

### Intra-sheath control wire anchoring and fixing pocket (cavity), and sheath control wire passageway (tunnel)

As illustratively described hereinabove, for the objective of improving anchoring and fixing of the distal end portion of a sheath flexible control wire to the inside of the sheath distal end portion, in exemplary embodiments of the sheath, the inside of the sheath distal end portion is also configured with an intra-sheath control wire anchoring and fixing pocket (or cavity). The intra-sheath control wire anchoring and fixing pocket (cavity) is configured (shaped and sized) so as to prevent, or at least minimize, possible latitudinal or/and (proximal-distal) longitudinal movement of a sheath control wire distal end portion that could lead to displacement thereof, or even exiting of the sheath control wire from inside the sheath distal end portion.

FIGs. 28 and 29 are schematic cross-sectional, and exploded cross-sectional, side views, respectively, of exemplary embodiments of the sheath 500, having a *cylindrical* proximal end portion 502pep, highlighting internal appearance and configuration of: (i) the two exemplary intra-sheath fluid (blood, water, air) flow passageways (tunnels) 512 and 513, (ii) an exemplary intra-sheath control wire anchoring and fixing pocket (cavity) 540 (542 + 544), and (iii) an exemplary sheath control wire passageway (tunnel) 550. In exemplary embodiments, the combination of (ii) and (iii) is also structured and functional as another exemplary intra-sheath fluid flow passageway (tunnel) inside the sheath distal end portion 506.

FIGs. 30 and 31 are schematic cross-sectional, and exploded cross-sectional, side views, respectively, of exemplary embodiments of the sheath 501, having a *conical* proximal end portion, highlighting internal appearance and configuration of: (i) the two exemplary intra-sheath fluid (blood, water, air) flow passageways (tunnels) 512 and 513, (ii) an exemplary intra-sheath control wire anchoring and fixing pocket (cavity) 540 (542 + 544), and (iii) an exemplary sheath control wire passageway (tunnel) 550. In exemplary embodiments, the combination of (ii) and (iii) is also structured and functional as another exemplary intra-sheath fluid flow passageway (tunnel) inside the sheath distal end portion 506.

FIGs. 32 and 33 are a schematic close-up perspective view, and a schematic close-up cross-sectional side view, respectively, of an exemplary embodiment of the sheath distal end portion 506 (including the cylindrical base 508 and the conical top 510 thereupon.

FIGs. 34 and 35 are schematic perspective views of an exemplary embodiment of the sheath 500, having a *cylindrical* proximal end portion 502pep, highlighting inclusion of an exemplary sheath control wire 560 extending along and through the inside of the sheath. FIGs. 36 and 37 are schematic perspective views of an exemplary embodiment of the sheath 501, having a *conical* proximal end portion 503pep, highlighting inclusion of an exemplary sheath control wire 560 extending along and through the inside of the sheath. FIGs. 38 and 39 are a schematic close-up perspective view, and a schematic close-up side view, respectively, of an exemplary embodiment of part of the sheath distal end portion 406, highlighting configuration of the sheath control wire 560 therein.

FIGs. 40 and 41 are schematic cross-sectional side views of exemplary embodiments of the sheaths 500 and 501, having a *cylindrical* proximal end portion [FIG. 40], and a *conical* proximal end portion [FIG. 41], respectively, highlighting the sheath control wire 560 anchored and fixedly connected to the inside of the sheath distal end portion 506. FIG. 42 is a schematic close-up cross-sectional side view of an exemplary embodiment of the sheath distal end portion 506, highlighting anchoring and fixed connection therein of the sheath control wire 560.

In exemplary embodiments, the intra-sheath control wire anchoring and fixing pocket (cavity) 540 (542 + 544) is tubular and includes two portions, namely, a first portion 542 and a second portion 544. In exemplary embodiments, the sheath control wire 560 has a distal end portion 560dep (for example, as shown in FIG. 42) that is located in the second portion 544 of the intra-sheath control wire anchoring and fixing pocket (cavity) 540. In exemplary embodiments, the intra-sheath control wire anchoring and fixing pocket (cavity) 540, and the sheath control wire 560, are configured (shaped and sized) relative to each other, so as to prevent, or at least minimize, possible latitudinal or/and (proximal-distal) longitudinal movements of the sheath control wire distal end portion 560dep that could lead to displacement thereof, or even exiting of the sheath control wire 560 from inside the sheath distal end portion 506.

In exemplary embodiments, the inner diameter of the first portion 542 is less than the inner diameter of the second portion 544. In exemplary embodiments, the sheath control wire distal end portion 560dep is positioned, anchored, and fixedly connected (attached) (e.g., using an adhesive or glue, or similar type of fixing material) to the inside of the second portion 544 (being the larger portion) of the intra-sheath control wire anchoring and fixing pocket (cavity) 540. Following anchoring and fixing of the sheath control wire distal end portion 560dep to the inside of the sheath distal end portion 506, such multi-portion (multi-stage) configuration of the intra-sheath control wire anchoring and fixing pocket (cavity) 540 restricts latitudinal and (proximal-distal) longitudinal movements of the sheath control wire 560 therein. Such is particularly relevant in exemplary embodiments wherein the outer diameter of the sheath control wire 560 is significantly less than the inner diameter of the first portion 542 (and therefore, even further less than the inner diameter of the second portion 544), whereby the possibility exits for the sheath control wire 560 to undergo latitudinal or/and (proximal-distal) longitudinal shifting type of movements inside the sheath distal end portion 506.

In exemplary embodiments, the inside of the proximal portion of the sheath distal end portion 506 is also configured with a sheath control wire passageway (tunnel), for example, sheath control wire passageway (tunnel) 550 (for example, as shown in FIGs. 28 - 31, 33, and 40 - 42). Exemplary sheath control wire passageway (tunnel) 550 is configured (shaped and sized) so as to provide a passageway (tunnel) within and along which a sheath control wire (for example, sheath control wire 560) is held, controlled, and guided, for example, by manual operation of a hole sealing device (for example, hole sealing device 106 disclosed in same applicant/assignee PCT Int'l. Pub. No. WO 2018/203237 A1). In exemplary embodiments, the sheath control wire passageway (tunnel) 550 has an inner diameter that is larger than the inner diameter of each of the first and second portions 542 and 544, respectively, of the intra-sheath control wire anchoring and fixing pocket (cavity) 540.

In exemplary embodiments of sheaths 500 and 501, the combination of the exemplary intra-sheath control wire anchoring and fixing pocket (cavity) 540 (542 + 544), and the exemplary sheath control wire passageway (tunnel) 550, is also structured and functional as another (i.e., a third) exemplary intra-sheath fluid (blood, water, air) flow passageway (tunnel) inside the sheath distal end portion 506. More specifically, the combination of the exemplary intra-sheath control wire anchoring and fixing pocket (cavity) 540 (542 + 544), and the exemplary sheath control wire passageway (tunnel) 550, is also structured and functional as another exemplary intra-sheath fluid (blood, water, air) flow passageway (tunnel) inside the sheath distal end portion 506, that facilitates *continuous* flow of blood vessel lumen fluids (blood, water, air) through the overall sheath 500 or 501, especially, flow of blood vessel lumen fluids that enter through the sheath (open) proximal end 502pe or 503pe, respectively, and exit through the sheath (open) distal end portion 506.

In exemplary embodiments wherein the sheath control wire 560 is anchored and fixedly connected to the inside of the sheath distal end portion 506 (for example, in the second portion 544 of the intra-sheath control wire anchoring and fixing pocket (cavity) 540), the presence of the sheath control wire 560 occupies space (volume) inside of such an additional exemplary intra-sheath fluid (blood, water, air) flow passageway (tunnel). Accordingly, such an additional exemplary intra-sheath fluid flow passageway (tunnel) may be limited in its effectiveness to (additionally) facilitate continuous flow of blood vessel lumen fluids through the overall sheath 500 or 501, compared to the effectiveness of the two exemplary intra-sheath fluid (blood, water, air) flow passageways (tunnels) 512 and 513 that more readily facilitate continuous flow of blood vessel lumen fluids through the overall sheath 500 or 501.

### Exemplary applications and implementations of the sheath

Exemplary embodiments of the herein disclosed sheath are particularly applicable for use in 'clampless' types of (end-to-side) surgical vascular anastomotic procedures, for performing coronary artery bypass grafting (CABG), for example, as disclosed in same applicant/assignee PCT Int'l. Pub. No. WO 2018/203237 A1.

FIGs. 43A and 43B are schematic diagrams of exemplary embodiments of implementing the sheath 400 [including a *single* exemplary intra-sheath fluid (blood, water, air) flow passageway (tunnel) 410 in the sheath distal end portion 406, and a *cylindrical* proximal end portion 402pep], with an exemplary hole sealing assembly, and an exemplary anastomotic hole generating device. FIGs. 44A and 44B are schematic diagrams of exemplary embodiments of implementing the sheath 401 [including a *single* exemplary intra-sheath fluid (blood, water, air) flow passageway (tunnel) 410 in the sheath distal end portion 406, and a *conical* proximal end portion 403pep], with an exemplary hole sealing assembly, and an exemplary anastomotic hole generating device.

FIGs. 45A and 45B are schematic diagrams of exemplary embodiments of implementing the sheath 500 [including a *plurality* of exemplary intra-sheath fluid (blood, water, air) flow passageways (tunnels) 512 and 513 in the sheath distal end portion 506, and a *cylindrical* proximal end portion 502pep], with an exemplary hole sealing assembly, and an exemplary anastomotic hole generating device. FIGs. 46A and 46B are schematic diagrams of exemplary embodiments of implementing the sheath 501 [including two exemplary intra-sheath fluid (blood, water, air) flow passageways (tunnels) 512 and 513 in the sheath distal end portion 506, and a *conical* proximal end portion 503pep], with an exemplary hole sealing assembly, and an exemplary anastomotic hole generating device.

In exemplary embodiments, the hole sealing assembly, and the anastomotic hole generating device, are the hole sealing assembly 112, and the anastomotic hole generating device 108, respectively, as disclosed in same applicant/assignee PCT Int'l. Pub. No. WO 2018/203237 A1. In exemplary embodiments, each sheath 400, 401, 500, and 501, includes a sheath control wire 460 or 560 whose distal end portion 460pep or 560dep, respectively, is anchored and fixedly connected to the inside of the respective sheath distal end portion 406 or 506 (for example, in the respective second portion 444 or 544 of the respective intra-sheath control wire anchoring and fixing pocket (cavity) 450 or 540, as described hereinabove). Outside of each sheath, the sheath control wire 460 or 560 is enclosed and held within, and extends along the length of, the cavity of a flexible tube 130 whose proximal end is fixedly connected to a manually operable hole sealing device (for example, hole sealing device 106 disclosed in same applicant/assignee PCT Int'l. Pub. No. WO 2018/203237 A1).

The exemplary hole sealing assembly 112 in a non-activated, collapsed configuration is present inside each sheath 400, 401, 500, and 501, as indicated by the dashed line arrow of reference number 112, but, is not visible in FIGs. 43A, 44A, 45A, and 46A. Each of FIGs. 43B, 44B, 45B, and 46B shows an exemplary embodiment of the hole sealing assembly 112 in an activated, self-expanded configuration, following removal of the sheath therefrom, for example, via manual operation of the hole sealing device with each respective sheath control wire 460 or 560.

In performing such surgical vascular anastomotic procedures, exemplary sheaths 400, 401, 500, and 501 may be used for externally covering, enclosing, and holding the (self-expandable) anastomotic hole sealing assembly 112 in a non-activated, collapsed configuration. For example, any of the exemplary sheaths (with the collapsed hole sealing assembly 112 therein) can be guided and positioned into a blood vessel lumen (e.g., of an aorta), by directing the sheath through a small (needle or syringe sized) hole or incision in a blood vessel wall. While inside the blood vessel lumen, the sheath is distally removed from the hole sealing assembly 112 so as to facilitate conversion and activation of the hole sealing assembly 112 from the collapsed configuration to an activated, self-expanded configuration. Thereafter, an anastomotic procedure (e.g., anastomosis) can be performed on the host blood vessel with a blood vessel graft, followed by proximally pulling the sheath back onto and over the distal end 112de of the hole sealing assembly 112, so as to return the hole sealing assembly 112 to a non-activated, collapsed configuration, prior to removal of the sheath (with the collapsed hole sealing assembly 112 therein) from the blood vessel, and ultimately, from the subject, followed by completing the anastomotic procedure.

According to such exemplary applications of the herein disclosed sheath, each intra-sheath fluid (blood, water, air) flow passageway (tunnel) 410, 512, 513, inside of the respective sheath distal end portion 406 or 506 facilitates *continuous* flow of blood vessel lumen fluids (blood, water, air) through the overall sheath, especially, flow of blood vessel lumen fluids that enter through the respective sheath (open) proximal end 402pe, 403pe, 502pe, or 503pe, and exit through the respective sheath (open) distal end portion 406 or 506. Even in scenarios that may include (temporary) accumulation of blood and thrombus formation inside the respective sheath 400, 401, 500, 501, when the sheath is proximally pulled back onto and over the distal end 112e of the the hole sealing assembly 112 (so as to return the hole sealing assembly 112 to its non-activated, collapsed configuration), entering of the hole sealing assembly 112 back into the sheath readily pushes and displaces the accumulated blood and thrombus out from inside the sheath, through each intra-sheath fluid flow passageway (tunnel) 410, 512, 513, and eventually through and out of the respective sheath distal end portion 406 or 506.

### Exemplary materials of construction and size dimensions of components, elements, and structural features, of the sheath for use in surgical vascular anastomotic procedures

### Sheath main portion (tubular member, shaft) 402, 403, 502, 503 [including the respective (cylindrical or conical) proximal end portion 402pep, 403pep, 502pep, 503pep]

### Materials of construction

In exemplary embodiments, the sheath main portion (402, 403, 502, 503) is made of a material selected from the group consisting of non-metallic materials, metallic materials, polymeric materials, composite materials, and a combination thereof. In exemplary embodiments, the sheath main portion (402, 403, 502, 503) is made of a non-metallic, polymeric material, for example, polyethylene (PET) plastic. In exemplary embodiments, the sheath main portion (402, 403, 502, 503) includes a porous or/and non-porous structure. In exemplary embodiments, the sheath main portion (402, 403, 502, 503) includes a porous structure (with holes or perforations), for example, in the form of a braid (having struts and holes or spaces therebetween), for example, made of a material selected from the group consisting of non-metallic materials, metallic materials, polymeric materials, composite materials, and a combination thereof. In exemplary embodiments, the sheath main portion (402, 403, 502, 503) is configured as a braid, for example, made of a non-metallic, polymeric material, such as polyethylene (PET) plastic.
- Sheath main portion (402, 403, 502, 503) external (outer) coating material (420, 520): polyether block amides, for example, Pebax^{®} polymeric resin.
- Sheath main portion (402, 403, 502, 503) internal (inner) coating material (425, 525): polytetrafluorethylenes (PTFEs), for example, Teflon^{®}.

### Size dimensions

- Sheath main portion (402, 403, 502, 503) outer diameter: range of 2 mm - 5 mm, for example, 3.2 mm.
- Sheath main portion (402, 403, 502, 503) inner diameter: range of 1.7 mm - 4.7 mm, for example, 2.9 mm.
- Sheath main portion wall (402w, 403w, 502w, 503w) thickness: range of 0.1 mm - 0.4 mm.
- Sheath main portion (402, 403, 502, 503) [proximal-distal] longitudinal length: range of 15 mm - 25 mm, for example, 19 mm.
- Conical proximal end portion (403pep, 503pep): [proximal-distal] longitudinal length: range of 0.5 mm - 4 mm, for example, 1.5 mm; apex (top) (403a, 503a) outer diameter: range of 2 mm - 5 mm, for example, 3.2 mm; base (bottom) (403b, 503b) outer diameter: range of 2 mm - 5 mm, for example 3.5 mm.
- Sheath main portion (402, 403, 502, 503) external (outer) coating material (420, 520) thickness: range of 30 microns - 300 microns, for example, 100 microns.
- Sheath main portion (402, 403, 502, 503) internal (inner) coating material (425, 525) thickness: range of 10 microns - 100 microns, for example, 30 microns.

### Sheath distal end portion 406, or 506 [including cylindrical base section 508 and conical top section 510]

### Materials of construction

In exemplary embodiments, the sheath distal end portion 406, or 506 (including the cylindrical base section 508 and the conical top section 510), is made of a material selected from the group consisting of non-metallic materials, metallic materials, polymeric materials, composite materials, and a combination thereof. In exemplary embodiments, the sheath distal end portion 506 cylindrical base section 508 and the conical top section 510 are made of a same material, or, alternatively, are made of different materials. In exemplary embodiments, the sheath distal end portion 406, or 506 (including the cylindrical base section 508 and the conical top section 510), is made of a single material, for example, a metallic material, such as stainless steel. In exemplary embodiments, the sheath distal end portion 406, or 506 (including the cylindrical base section 508 and the conical top section 510), is made of a single material, for example, a non-metallic, polymeric material, such as PEEK (polyether ether ketone).

### Size dimensions

- Sheath distal end portion (406) overall [proximal-distal] longitudinal length, or (506) overall [proximal-distal] longitudinal length = cylindrical base section (508) length + conical top section (510) length: range of 2 mm - 6 mm, for example, 4 mm.
- Cylindrical base section (508) diameter: range of 2 mm - 5 mm, for example, 3.2 mm.
- Conical top section (510), apex (top) 510a diameter: range of 0.5 mm - 2 mm.
- Conical top section (510), base 510b diameter: range of 2 mm - 5 mm, for example, 3.2 mm.
- Conical top section (510) [proximal-distal] longitudinal length: range of 1 mm - 3 mm, for example, 2 mm.

### Intra-sheath fluid (blood, water, air) flow passageways (tunnels) (410, 512, 513)

- diameter: range of 0.8 mm - 1.0 mm, for example, 1.0 mm.
- length (406a - 406b, 514a - 514b, 516a - 516b): range of 2 mm - 5 mm, for example, 3.2 mm.

### Intra-sheath control wire anchoring and fixing pocket (cavity) 440 [first portion 442 + second portion 4441, or 540 [first portion 542 + second portion 544]

- first portion 442, 542 diameter: range of 0.2 mm - 1 mm.
- first portion 442, 542 [proximal-distal] longitudinal length: 0.5 mm - 6 mm.
- second portion 444, 544 diameter: range of 1 mm - 3 mm.
- second portion 444, 544 [proximal-distal] longitudinal length: 1 mm - 5 mm.

### Sheath control wire passageway (tunnel) 450, 550

- diameter: range of 1 mm - 3 mm.
- [proximal-distal] longitudinal length: 1 mm - 6 mm.

In exemplary embodiments, the sheath flexible control wire 460 or 560 is made of a material selected from the group consisting of nitinol, cobalt, and chrome, and has a diameter in a range of 0.1 mm - 0.5 mm, for example, 0.3 mm.

Each of the following terms written in singular grammatical form: 'a', 'an', and 'the', as used herein, means 'at least one', or 'one or more'. Use of the phrase 'one or more' herein does not alter this intended meaning of 'a', 'an', or 'the'. Accordingly, the terms 'a', 'an', and 'the', as used herein, may also refer to, and encompass, a plurality of the stated entity or object, *unless otherwise specifically defined or stated herein,* or, *unless the context clearly dictates otherwise.* For example, the phrases: 'a unit', 'a device', 'an assembly', 'a mechanism', 'a component', 'an element', and 'a step or procedure', as used herein, may also refer to, and encompass, a plurality of units, a plurality of devices, a plurality of assemblies, a plurality of mechanisms, a plurality of components, a plurality of elements, and, a plurality of steps or procedures, respectively.

Each of the following terms: 'includes', 'including', 'has', 'having', 'comprises', and 'comprising', and, their linguistic / grammatical variants, derivatives, or/and conjugates, as used herein, means 'including, but not limited to', and is to be taken as specifying the stated component(s), feature(s), characteristic(s), parameter(s), integer(s), or step(s), and does not preclude addition of one or more additional component(s), feature(s), characteristic(s), parameter(s), integer(s), step(s), or groups thereof.

Each of the phrases 'consisting of and 'consists of, as used herein, means 'including and limited to'.

The phrase 'consisting essentially of', as used herein, means that the stated entity or item (system, system unit, system sub-unit, device, assembly, sub-assembly, mechanism, structure, component, element, or, peripheral equipment, utility, accessory, or material, method or process, step or procedure, sub-step or sub-procedure), which is an entirety or part of an exemplary embodiment of the disclosed invention, or/and which is used for implementing an exemplary embodiment of the disclosed invention, may include at least one additional 'feature or characteristic' being a system unit, system sub-unit, device, assembly, sub-assembly, mechanism, structure, component, or element, or, peripheral equipment, utility, accessory, or material, step or procedure, sub-step or sub-procedure), but only if each such additional 'feature or characteristic' does not materially alter the basic novel and inventive characteristics or special technical features, of the claimed entity or item.

The term 'method', as used herein, refers to a single step, procedure, manner, means, or/and technique, or a sequence, set, or group of two or more steps, procedures, manners, means, or/and techniques, for accomplishing or achieving a given task or action. Any such herein disclosed method, in a non-limiting manner, may include one or more steps, procedures, manners, means, or/and techniques, that are known or readily developed from one or more steps, procedures, manners, means, or/and techniques, previously taught about by practitioners in the relevant field(s) and art(s) of the herein disclosed invention. In any such herein disclosed method, in a non-limiting manner, the stated or presented *sequential order* of one or more steps, procedures, manners, means, or/and techniques, is not limited to that specifically stated or presented sequential order, for accomplishing or achieving a given task or action, *unless otherwise specifically defined or stated herein,* or, *unless the context clearly dictates otherwise.* Accordingly, in any such herein disclosed method, in a non-limiting manner, there may exist one or more alternative sequential orders of the same steps, procedures, manners, means, or/and techniques, for accomplishing or achieving a same given task or action, while maintaining same or similar meaning and scope of the herein disclosed invention.

Throughout this disclosure, a numerical value of a parameter, feature, characteristic, object, or dimension, may be stated or described in terms of a numerical range format. Such a numerical range format, as used herein, illustrates implementation of some exemplary embodiments of the invention, and does not inflexibly limit the scope of the exemplary embodiments of the invention. Accordingly, a stated or described numerical range also refers to, and encompasses, all possible sub-ranges and individual numerical values (where a numerical value may be expressed as a whole, integral, or fractional number) within that stated or described numerical range. For example, a stated or described numerical range 'from 1 to 6' also refers to, and encompasses, all possible sub-ranges, such as 'from 1 to 3', 'from 1 to 4', 'from 1 to 5', 'from 2 to 4', 'from 2 to 6', 'from 3 to 6', etc., and individual numerical values, such as '1', '1.3', '2', '2.8', '3', '3.5', '4', '4.6', '5', '5.2', and '6', within the stated or described numerical range of 'from 1 to 6'. This applies regardless of the numerical breadth, extent, or size, of the stated or described numerical range.

Moreover, for stating or describing a numerical range, the phrase 'in a range of *between* about a first numerical value *and* about a second numerical value', is considered equivalent to, and meaning the same as, the phrase 'in a range of *from* about a first numerical value *to* about a second numerical value', and, thus, the two equivalently meaning phrases may be used interchangeably. For example, for stating or describing the numerical range of room temperature, the phrase 'room temperature refers to a temperature in a range of between about 20 °C and about 25 °C', is considered equivalent to, and meaning the same as, the phrase 'room temperature refers to a temperature in a range of from about 20 °C to about 25 °C'.

The term 'about', as used herein, refers to ± 10 % of the stated numerical value.

The phrase 'operatively connected', as used herein, equivalently refers to the corresponding synonymous phrases 'operatively joined', and 'operatively attached'. These phrases, as used herein, mean that the described or/and shown entities are configured 'connected' to each other, in an 'operative' (ready-for-operation / ready-for-use) manner. Such operative connection, operative joint, or operative attachment, between or among the entities is according to one type, or a plurality of types, of a mechanical (physical, structural), or/and an electrical, or/and an electronic, or/and an electro-mechanical, connection or connections, involving one or more corresponding type(s) or kind(s) of mechanical (physical, structural), or/and electrical, or/and electronic, or/and electro-mechanical, equipment and components. Optionally, such operative connection, operative joint, or operative attachment, between or among the entities, may include, or may involve, one or more type(s) or kind(s) of computerized hardware or/and software equipment and components.

The phrase 'operably connectable', as used herein, equivalently refers to the corresponding synonymous phrases 'operably joinable to', and 'operably attachable to'. These phrases, as used herein, mean that the described or/and shown entities are configured 'connectable' to each other (i.e., capable of being connected to each other, having ability to be connected to each other, or having potential to be connected to each other), for subsequently forming an 'operative connection', an 'operative joint', or an 'operative attachment', between or among the entities. Such operable connectability, operable joinability, or operable attachability, between or among the entities is according to one type, or a plurality of types, of a mechanical (physical, structural), or/and an electrical, or/and an electronic, or/and an electro-mechanical, connection or connections, involving one or more corresponding type(s) or kind(s) of mechanical (physical, structural), or/and electrical, or/and electronic, or/and electro-mechanical, equipment and components. Optionally, such operable connectability, operable joinability, or operable attachability, between or among the entities, may include, or may involve, one or more type(s) or kind(s) of computerized hardware or/and software equipment and components.

It is to be fully understood that certain aspects, characteristics, and features, of the invention, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub-combination in the context or format of a single embodiment, may also be illustratively described and presented in the context or format of a plurality of separate embodiments.

Although the invention has been illustratively described and presented by way of specific exemplary embodiments, and examples thereof, it is evident that many alternatives, modifications, or/and variations, thereof, will be apparent to those skilled in the art. Accordingly, it is intended that all such alternatives, modifications, or/and variations, are encompassed by the broad scope of the appended claims.

All publications, patents, and or/and patent applications, cited or referred to in this disclosure are herein incorporated in their entirety by reference into the specification, to the same extent as if each individual publication, patent, or/and patent application, was specifically and individually indicated to be incorporated herein by reference. In addition, citation or identification of any reference in this specification shall not be construed or understood as an admission that such reference represents or corresponds to prior art of the present invention. To the extent that section headings are used, they should not be construed as necessarily limiting.

## Claims

1. A sheath (400, 401, 500, 501), for externally covering, enclosing, and holding a self-expandible anastomotic hole sealing assembly (112), for use in performing surgical vascular anastomotic procedures, the sheath (400, 401, 500, 501) comprising:
a tubular sheath main portion (402, 403, 502, 503) configured as a tubular member and including a sheath main portion distal end (402de, 403de, 502de, 503de) and a sheath main portion proximal end (402pe, 403pe, 502pe, 503pe), said sheath main portion proximal end (402pe, 403pe, 502pe, 503pe) is open, and said tubular sheath main portion (402, 403, 502, 503) is configured for covering, enclosing, and holding the hole sealing assembly (112) in a collapsed configuration;
and
a sheath distal end portion (406, 506), continuous with said sheath main portion distal end (402de, 403de, 502de, 503de), and including an intra-sheath fluid flow passageway (410) that extends and passes entirely through the middle or center, proximal-distal, longitudinal axis inside of said sheath distal end portion (406, 506), via two diametrically opposed, and centrally, located parts (406a, 406b) of said sheath distal end portion (406, 506), and wherein said sheath distal end portion (406, 506) also includes an intra-sheath control wire anchoring and fixing pocket (440, 540) that is tubular and includes two portions, a first portion (442, 542) and a second portion (444, 544), wherein the inner diameter of said first portion (442, 542) is less than the inner diameter of said second portion (444, 544);
whereby each said intra-sheath fluid flow passageway (410, 512, 513) facilitates continuous flow of blood vessel lumen fluids through the sheath (400, 401, 500, 501);
**characterised in that** the sheath (400, 401, 500, 501) further includes a sheath control wire (460, 560), longitudinally extending along and through the inside of the sheath (400, 401, 500, 501), and being anchored and fixedly connected to the inside of said second portion (444, 544) of said intra-sheath control wire anchoring and fixing pocket (440, 540), whereby said fixed sheath control wire (460, 560) is configured to distally remove the sheath (400, 401, 500, 501) from the hole sealing assembly (112) in the collapsed configuration, so as to facilitate conversion of the hole sealing assembly (112) to a self-expanded configuration, and said fixed sheath control wire (460, 560) is configured to proximally pull the sheath (400, 401, 500, 501) back onto and over the hole sealing assembly (112), so as to return the hole sealing assembly (112) to the collapsed configuration, prior to removal of the sheath (400, 401, 500, 501) from a blood vessel, and from a subject undergoing an anastomotic procedure.

2. The sheath (400, 401, 500, 501) of claim 1, wherein said sheath main portion (402, 403, 502, 503) and said sheath distal end portion (406, 506) are configured as a single, monolithic structure, wherein said sheath main portion (402, 403, 502, 503) and said sheath distal end portion (406, 506) are integrally formed as a single continuous structure.

3. The sheath (400, 401, 500, 501) of claim 1, wherein said sheath main portion (402, 403, 502, 503) and said sheath distal end portion (406, 506) are configured as two individual, operably connectable, structures.

4. The sheath (400, 401, 500, 501) of claim 3, wherein said sheath distal end portion (406, 506) is configured with a proximal end portion (406pep, 517) that closely fits into the tubular distal end portion ( 402dep, 403dep, 518) of said sheath main portion (402, 403, 502, 503).

5. The sheath (400, 401, 500, 501) of claim 1, wherein said sheath main portion (402, 403, 502, 503) has a conical proximal end portion (403pep, 503pep) that includes said sheath main portion open proximal end (403pe, 503pe).

6. The sheath (400, 401, 500, 501) of claim 1, wherein said sheath distal end portion (406, 506) is also configured with a sheath control wire passageway (450, 550).

7. The sheath (400, 401, 500, 501) of claim 6, wherein said sheath control wire passageway (450, 550) has an inner diameter that is larger than the inner diameter of each of said first (442, 542) and second (444, 544) portions of said intra-sheath control wire anchoring and fixing pocket (440, 540).

8. The sheath (400, 401, 500, 501) of claim 1, wherein at least one said intra-sheath fluid flow passageway (410) extends and passes entirely through a non-middle or off-center, proximal-distal, longitudinal axis inside of said sheath distal end portion (406, 506), via two oppositely, diametrically opposed, and non-centrally, located parts (406a, 406b) of said sheath distal end portion (406, 506).

9. The sheath (400, 401, 500, 501) of claim 1, wherein said sheath distal end portion (406, 506) further includes at least one said intra-sheath fluid flow passageway (512, 513) radially, orthogonally to proximal - distal directions, extends and passes entirely through the inside of said sheath distal end portion (406, 506), via a pair of two oppositely located, diametrically opposed, parts (514a, 514b, 516a, 516b) of the circumferential periphery surface (506cps) of said sheath distal end portion (406, 506).

10. The sheath (400, 401, 500, 501) of claim 1, wherein said sheath distal end portion (406, 506) is configured as a member having a cylindrical base section (508) and a conical top section (510) thereupon, and wherein at least one said intra-sheath fluid flow passageway (410, 512, 513) extends and passes entirely through the inside of said cylindrical base section (508) of said sheath distal end portion (406, 506).

11. The sheath (400, 401, 500, 501) of claim 10, wherein said intra-sheath fluid flow passageway (512, 513) radially, orthogonally to proximal - distal directions, extends and passes entirely through the inside of said cylindrical base section (508) of said sheath distal end portion (406, 506).

12. The sheath (400, 401, 500, 501) of claim 11, wherein said intra-sheath fluid flow passageway (512, 513) radially, orthogonally to proximal - distal directions, extends and passes entirely through the inside of said conical top section (510) of said sheath distal end portion (406, 506).

13. The sheath (400, 401, 500, 501) of claim 1, wherein said sheath main portion (402, 403, 502, 503) is made of a material selected from the group consisting of non-metallic materials, metallic materials, polymeric materials, composite materials, and a combination thereof.

## Patentansprüche

1. Eine Hülle (400, 401, 500, 501) zum äußeren Abdecken, Umschließen und Halten einer selbstexpandierenden Anastomosenloch-Dichtungsanordnung (112) zur Verwendung bei der Durchführung chirurgischer Gefäßanastomosenverfahren, wobei die Hülle (400, 401, 500, 501) umfasst:
einen röhrenförmigen Hauptabschnitt der Hülle (402, 403, 502, 503), der als röhrenförmiges Element ausgebildet ist und ein distales Ende des Hauptabschnitts der Hülle (402de, 403de, 502de, 503de) und ein proximales Ende (402pe, 403pe, 502pe, 503pe) aufweist, wobei das proximale Ende (402pe, 403pe, 502pe, 503pe) des Hauptabschnitts der Hülle offen ist und der röhrenförmige Hauptabschnitt (402, 403, 502, 503) so konfiguriert ist, dass er die Loch-Dichtungsanordnung (112) in einer kollabierten Konfiguration abdeckt, umschließt und hält;
und
ein distaler Endabschnitt (406, 506) der Hülle, der mit dem distalen Ende des Hauptabschnitts (402de, 403de, 502de, 503de) der Hülle zusammenhängt und einen Fluidströmungskanal (410) innerhalb der Hülle umfasst, der sich vollständig durch die mittlere oder zentrale, proximal-distale, Längsachse innerhalb des distalen Endabschnitts (406, 506) der Hülle erstreckt und durch diese vollständig hindurch verläuft, und zwar durch zwei diametral gegenüberliegende und zentral angeordnete Teile (406a, 406b) des distalen Endabschnitts (406, 506) der Hülle, und wobei der distale Endabschnitt (406, 506) der Hülle außerdem eine Verankerungs- und Befestigungstasche (440, 540) für einen Steuerdraht innerhalb der Hülle umfasst, die röhrenförmig ist und zwei Abschnitte umfasst, einen ersten Abschnitt (442, 542) und einen zweiten Abschnitt (444, 544), wobei der Innendurchmesser des ersten Abschnitts (442, 542) kleiner ist als der Innendurchmesser des zweiten Abschnitts (444, 544);
wobei jeder der genannte Flüssigkeitsströmungskanal (410, 512, 513) innerhalb der Hülle einen kontinuierlichen Fluss von Flüssigkeiten aus dem Lumen des Blutgefäßes durch die Hülle (400, 401, 500, 501) ermöglicht;
**dadurch gekennzeichnet, dass** die Hülle (400, 401, 500, 501) ferner einen Hüllensteuerdraht (460, 560) umfasst, der sich in Längsrichtung entlang und durch das Innere der Hülle (400, 401, 500, 501) erstreckt und der im Inneren des zweiten Abschnitts (444, 544) der Verankerungs- und Befestigungstasche (440, 540) für den Steuerdraht innerhalb der Hülle verankert und fest mit diesem verbunden ist, wobei der fixierte Hüllensteuerdraht (460, 560) dazu konfiguriert ist, die Hülle (400, 401, 500, 501) distal aus der Loch-Dichtungsanordnung (112) in der kollabierten Konfiguration zu entfernen, um die Umwandlung der Loch-Dichtungsanordnung (112) in eine selbstexpandierte Konfiguration zu erleichtern, und der fixierte Hüllensteuerdraht (460, 560) dazu konfiguriert ist, die Hülle (400, 401, 500, 501) proximal zurück auf und über die Loch-Dichtungsanordnung (112) zu ziehen, um die Loch-Dichtungsanordnung (112) in die kollabierte Konfiguration zurückzubringen, bevor die Hülle (400, 401, 500, 501) aus einem Blutgefäß und aus einem Patienten, der sich einem Anastomoseprozess unterzieht, entfernt wird.

2. Die Hülle (400, 401, 500, 501) nach Anspruch 1, wobei der Hauptabschnitt (402, 403, 502, 503) der Hülle und der distale Endabschnitt (406, 506) der Hülle als eine einzige, monolithische Struktur ausgebildet sind, wobei der Hauptabschnitt (402, 403, 502, 503) der Hülle und der distale Endabschnitt (406, 506) der Hülle integral als eine einzige durchgehende Struktur ausgebildet sind.

3. Die Hülle (400, 401, 500, 501) nach Anspruch 1, wobei der Hauptabschnitt (402, 403, 502, 503) der Hülle und der distale Endabschnitt (406, 506) der Hülle als zwei einzelne, funktionsfähig verbindbare Strukturen ausgebildet sind.

4. Die Hülle (400, 401, 500, 501) nach Anspruch 3, wobei der distale Endabschnitt (406, 506) der Hülle mit einem proximalen Endabschnitt (406pep, 517) konfiguriert ist, der in dem röhrenförmigen distalen Endabschnitt (402dep, 403dep, 518) des Hauptabschnitts (402, 403, 502, 503) der Hülle eng anliegt.

5. Die Hülle (400, 401, 500, 501) nach Anspruch 1, wobei der Hauptabschnitt (402, 403, 502, 503) der Hülle einen konischen proximalen Endabschnitt (403pep, 503pep) aufweist, der das offene proximale Ende (403pe, 503pe) des Hauptabschnitts der Hülle umfasst.

6. Die Hülle (400, 401, 500, 501) nach Anspruch 1, wobei der distale Endabschnitt (406, 506) der Hülle ebenfalls mit einem Hüllensteuerdrahtdurchgang (450, 550) konfiguriert ist.

7. Die Hülle (400, 401, 500, 501) nach Anspruch 6, wobei der Hüllensteuerdrahtdurchgang (450, 550) einen Innendurchmesser aufweist, der größer ist als der Innendurchmesser jedes der ersten (442, 542) und zweiten (444, 544) Abschnitte der Verankerungs- und Befestigungstasche (440, 540) für den Steuerdraht innerhalb der Hülle.

8. Die Hülle (400, 401, 500, 501) nach Anspruch 1, wobei sich der mindestens eine der Flüssigkeitsströmungskanal (410) innerhalb der Hülle vollständig durch eine nicht mittige oder nicht zentrale, proximal-distale Längsachse innerhalb des distalen Endabschnitts (406, 506) der Hülle erstreckt und verläuft, und zwar durch zwei einander gegenüberliegende, diametral gegenüberliegende und nicht zentral angeordnete Teilen (406a, 406b) des distalen Endabschnitts (406, 506) der Hülle.

9. Die Hülle (400, 401, 500, 501) nach Anspruch 1, wobei der distale Endabschnitt (406, 506) der Hülle ferner mindestens einen genannten Flüssigkeitsdurchgangskanal (512, 513) innerhalb der Hülle umfasst, der sich radial, orthogonal zu den proximalen und distalen Richtungen, vollständig durch das Innere des distalen Endabschnitts (406, 506) der Hülle erstreckt und diesen durchläuft, durch ein Paar von zwei gegenüberliegend angeordneten, diametral gegenüberliegenden Teilen (514a, 514b, 516a, 516b) der Umfangsperipherieoberfläche (506cps) des distalen Endabschnitts (406, 506) der Hülle.

10. Die Hülle (400, 401, 500, 501) nach Anspruch 1, wobei der distale Endabschnitt (406, 506) der Hülle als ein Element mit einem zylindrischen Basisabschnitt (508) und einem darauf befindlichen konischen oberen Abschnitt (510) ausgebildet ist und wobei sich der mindestens eine Fluidströmungskanal (410, 512, 513) innerhalb der Hülle (410, 512, 513) vollständig durch das Innere des zylindrischen Basisabschnitts (508) des distalen Endabschnitts (406, 506) der Hülle erstreckt und diesen durchläuft.

11. Die Hülle (400, 401, 500, 501) nach Anspruch 10, wobei sich der Flüssigkeitsströmungskanal (512, 513) innerhalb der Hülle radial, orthogonal zu den proximalen und distalen Richtungen, erstreckt und vollständig durch das Innere des zylindrischen Basisabschnitts (508) des distalen Endabschnitts (406, 506) der Hülle verläuft.

12. Die Hülle (400, 401, 500, 501) nach Anspruch 11, wobei sich der Flüssigkeitsströmungskanal (512, 513) innerhalb der Hülle radial, orthogonal zu den proximalen und distalen Richtungen, erstreckt und vollständig durch das Innere des konischen oberen Abschnitts (510) des distalen Endabschnitts (406, 506) der Hülle verläuft.

13. Die Hülle (400, 401, 500, 501) nach Anspruch 1, wobei der Hauptabschnitt (402, 403, 502, 503) der Hülle aus einem Material hergestellt ist, das aus der Gruppe ausgewählt ist, die aus nichtmetallischen Materialien, metallischen Materialien, polymeren Materialien, Verbundmaterialien und einer Kombination davon besteht.

## Revendications

1. Gaine (400, 401, 500, 501) destinée à recouvrir, enfermer et maintenir de manière externe un ensemble auto-expansible de scellement de trou anastomotique (112), à utiliser dans la réalisation de procédures chirurgicales d'anastomose vasculaire, la gaine (400, 401, 500, 501) comprenant :
une partie principale de gaine tubulaire (402, 403, 502, 503) configurée comme un élément tubulaire et comprenant une extrémité distale de partie principale de gaine (402de, 403de, 502de, 503de) et une extrémité proximale de partie principale de gaine (402pe, 403pe, 502pe, 503pe), ladite extrémité proximale de partie principale de gaine (402pe, 403pe, 502pe, 503pe) étant ouverte, et ladite partie principale de gaine tubulaire (402, 403, 502, 503) est configurée pour recouvrir, enfermer et maintenir l'ensemble d'étanchéité de trou (112) dans une configuration repliée;
et
une partie d'extrémité distale de la gaine (406, 506), continue avec ladite extrémité distale de la partie principale de la gaine (402de, 403de, 502de, 503de), et comprenant un passage d'écoulement de fluide intra-gaine (410) qui s'étend et passe entièrement à travers le milieu ou le centre, proximal-distal, l'axe longitudinal à l'intérieur de ladite partie d'extrémité distale de la gaine (406, 506), via deux parties diamétralement opposées et situées au centre (406a, 406b) de ladite partie d'extrémité distale de la gaine (406, 506), et dans laquelle ladite partie d'extrémité distale de la gaine (406, 506) comprend également une poche d'ancrage et de fixation du fil de commande intra-gaine (440, 540) qui est tubulaire et comprend deux parties, une première partie (442, 542) et une deuxième partie (444, 544), dans laquelle le diamètre intérieur de ladite première partie (442, 542) est inférieur au diamètre intérieur de ladite deuxième partie (444, 544) ;
chaque passage d'écoulement de fluide intra-gaine (410, 512, 513) facilitant l'écoulement continu des fluides de la lumière des vaisseaux sanguins à travers la gaine (400, 401, 500, 501);
**caractérisé en ce que** la gaine (400, 401, 500, 501) comprend en outre un fil de commande de gaine (460, 560), s'étendant longitudinalement le long et à travers l'intérieur de la gaine (400, 401, 500, 501), et étant ancré et relié de manière fixe à l'intérieur de ladite deuxième partie (444, 544) de ladite poche d'ancrage et de fixation du fil de commande intra-gaine (440, 540), de sorte que ledit fil de commande de gaine fixe (460, 560) est configuré pour retirer de manière distale la gaine (400, 401, 500, 501) de l'ensemble d'étanchéité de trou (112) dans la configuration repliée, de manière à faciliter la conversion de l'ensemble d'étanchéité de trou (112) en une configuration auto-expansée, et ledit fil de commande de gaine fixe (460, 560) est configuré pour tirer de manière proximale la gaine (400, 401, 500, 501) vers l'arrière sur et au-dessus de l'ensemble d'obturation de trou (112), de manière à ramener l'ensemble d'obturation de trou (112) dans la configuration repliée, avant le retrait de la gaine (400, 401, 500, 501) d'un vaisseau sanguin et d'un sujet subissant une procédure d'anastomose.

2. La gaine (400, 401, 500, 501) selon la revendication 1, dans laquelle ladite partie principale de la gaine (402, 403, 502, 503) et ladite partie d'extrémité distale de la gaine (406, 506) sont configurées comme une structure monolithique unique, dans laquelle ladite partie principale de la gaine (402, 403, 502, 503) et ladite partie d'extrémité distale de la gaine (406, 506) sont formées d'un seul tenant comme une structure continue unique.

3. La gaine (400, 401, 500, 501) selon la revendication 1, dans laquelle ladite partie principale de gaine (402, 403, 502, 503) et ladite partie d'extrémité distale de la gaine (406, 506) sont configurées comme deux structures individuelles pouvant être reliées de manière opérationnelle.

4. La gaine (400, 401, 500, 501) selon la revendication 3, dans laquelle ladite partie d'extrémité distale de la gaine (406, 506) est configurée avec une partie d'extrémité proximale (406pep, 517) qui s'adapte étroitement dans la partie d'extrémité distale tubulaire (402dep, 403dep, 518) de ladite partie principale de gaine (402, 403, 502, 503).

5. La gaine (400, 401, 500, 501) selon la revendication 1, dans laquelle ladite partie principale de gaine (402, 403, 502, 503) comporte une partie d'extrémité proximale conique (403pep, 503pep) qui comprend ladite extrémité proximale ouverte (403pe, 503pe) de la partie principale de la gaine.

6. La gaine (400, 401, 500, 501) selon la revendication 1, dans laquelle ladite partie d'extrémité distale de la gaine (406, 506) est également configurée avec un passage pour fil de commande de gaine (450, 550).

7. La gaine (400, 401, 500, 501) de la revendication 6, dans lequel ledit passage de fil de commande de gaine (450, 550) a un diamètre intérieur qui est plus grand que le diamètre intérieur de chacune desdites première (442, 542) et deuxième (444, 544) parties de ladite poche d'ancrage et de fixation de fil de commande intra-gaine (440, 540).

8. La gaine (400, 401, 500, 501) selon la revendication 1, dans laquelle au moins un desdits passages d'écoulement de fluide intra-gaine (410) s'étend et passe entièrement à travers un axe longitudinal non médian ou décentré, proximal-distal, à l'intérieur de ladite partie d'extrémité distale de la gaine (406, 506), via deux parties opposées, diamétralement opposées et non centrées (406a, 406b) de ladite partie d'extrémité distale de la gaine (406, 506).

9. La gaine (400, 401, 500, 501) de la revendication 1, dans laquelle ladite partie d'extrémité distale de la gaine (406, 506) comprend en outre au moins un dit passage d'écoulement de fluide intra-gaine (512, 513) radialement, orthogonalement aux directions proximale et distale, s'étend et traverse entièrement l'intérieur de ladite partie d'extrémité distale de la gaine (406, 506), via une paire de deux parties (514a, 514b, 516a, 516b) situées de manière opposée, diamétralement opposées, de la surface périphérique circonférentielle (506cps) de ladite partie d'extrémité distale de la gaine (406, 506).

10. La gaine (400, 401, 500, 501) selon la revendication 1, dans laquelle ladite partie d'extrémité distale de la gaine (406, 506) est configurée comme un élément comportant une section de base cylindrique (508) et une section supérieure conique (510) sur celle-ci, et dans laquelle au moins un desdits passages d'écoulement de fluide intra-gaine (410, 512, 513) s'étend et passe entièrement à l'intérieur de ladite section de base cylindrique (508) de ladite partie d'extrémité distale de gaine (406, 506).

11. La gaine (400, 401, 500, 501) de la revendication 10, dans laquelle ledit passage d'écoulement de fluide intra-gaine (512, 513) s'étend radialement, orthogonalement aux directions proximale et distale, et traverse entièrement l'intérieur de ladite section de base cylindrique (508) de ladite partie d'extrémité distale de gaine (406, 506).

12. La gaine (400, 401, 500, 501) de la revendication 11, dans lequel ledit passage d'écoulement de fluide intra-gaine (512, 513) s'étend radialement, orthogonalement aux directions proximale et distale, et traverse entièrement l'intérieur de ladite section supérieure conique (510) de ladite partie d'extrémité distale de gaine (406, 506).

13. La gaine (400, 401, 500, 501) selon la revendication 1, dans laquelle ladite partie principale de gaine (402, 403, 502, 503) est constituée d'un matériau choisi parmi le groupe comprenant les matériaux non métalliques, les matériaux métalliques, les matériaux polymères, les matériaux composites et une combinaison de ceux-ci.
